# EUROPEAN PATENT APPLICATION

(11) **EP 4 470 474 A1**
(43) Date of publication of application: **04.12.2024**
(21) Application number: 22923997.5
(22) Date of filing: 04.10.2022
(51) Int. Cl.: A61B 10/00, A45D 34/02, A61L 9/12

(54) **ODOR GENERATION DEVICE, OLFACTORY TEST OR OLFACTORY TRAINING SYSTEM, OLFACTORY PRESENTATION NEURODEGENERATIVE DISEASE PREVENTION AND/OR TREATMENT DEVICE, AND ODOR EXPERIENCE DEVICE**

(30) Priority: 25.01.2022 JP 2022009655; 10.06.2022 JP 2022094382
(71) Applicant: Sony Group Corporation, Tokyo 108-0075 (JP)
(72) Inventor: INOUE, Yukito, Tokyo 108-0075 (JP); FUJITA, Shuji, Tokyo 108-0075 (JP); KIHARA, Hirotaka, Tokyo 108-0075 (JP); OOTANI, Shinya, Tokyo 108-0075 (JP)
(74) Representative: D Young & Co LLP
(86) International application number: PCT/JP2022/037184
(87) International publication number: WO 2023/145139

(57) **Abstract**

To provide a technique capable of delivering an odor component to a desired position.

The present technology first provides an odor generation device including: a cartridge that holds a tubular body containing an odor component; a release unit that releases the odor component to an outside; and a placement drive unit that, among a plurality of the cartridges, places a specific cartridge near the release unit. Furthermore, the present technology also provides an olfactory examination or olfactory training system, an olfactory presentation neurodegenerative disease prevention and/or treatment device, and an odor experience device including the odor generation device and an information processing device that controls the placement drive unit.

## Description

### TECHNICAL FIELD

The present technology relates to an odor generation device, an olfactory examination or olfactory training system, an olfactory presentation neurodegenerative disease prevention and/or treatment device, and an odor experience device. More specifically, the present invention relates to an odor generation device, an olfactory examination or olfactory training system, an olfactory presentation neurodegenerative disease prevention and/or treatment device, and an odor experience device capable of delivering an odor component to a desired position.

### BACKGROUND ART

Conventionally, a technology has been proposed in which air is supplied to an odor holding body that holds an odor and the odor is released by a flow of the air to provide the odor to a user.

For example, Patent Document 1 proposes an aroma device including an aroma head capable of releasing a gas containing an aroma substance at a nose tip of a user and exhausting air for deodorization. Furthermore, Patent Document 1 proposes that in the aroma device, a pipe for supplying an odorless gas used for releasing a gas containing an aroma substance and a pipe for intake air for performing deodorization are integrated with a free arm that holds the aroma head in an air position.

### CITATION LIST

### PATENT DOCUMENT

Patent Document 1: Japanese Patent Application Laid-Open No. 2005-304608

### SUMMARY OF THE INVENTION

### PROBLEMS TO BE SOLVED BY THE INVENTION

However, in the conventional odor generation device, depending on the odor and its concentration, the odor adheres to a member constituting the odor generation device and cannot be taken for a certain period of time. Therefore, it is necessary to prevent the odor from adhering to the member constituting the odor generation device as much as possible by bringing a release opening of the odor component close to the nose tip of the user.

Therefore, a main object of the present technology is to provide a technology capable of delivering an odor component to a desired position.

### SOLUTIONS TO PROBLEMS

The present technology first provides an odor generation device including: a cartridge that holds a tubular body containing an odor component; a release unit that releases the odor component to an outside; and a placement drive unit that, among a plurality of the cartridges, places a specific cartridge near the release unit.

Furthermore, the present technology also provides an olfactory examination or olfactory training system including: an odor generation device including, a cartridge that holds a tubular body containing an odor component, a release unit that releases the odor component to an outside, and a placement drive unit that, among a plurality of the cartridges, places a specific cartridge near the release unit; and an information processing device that controls the placement drive unit.

Moreover, the present technology also provides an olfactory presentation neurodegenerative disease prevention and/or treatment device including: an odor generation device including, a cartridge that holds a tubular body containing an odor component, a release unit that releases the odor component to an outside, and a placement drive unit that, among a plurality of the cartridges, places a specific cartridge near the release unit; and an information processing device that controls the placement drive unit.

In addition, the present technology also provides an odor experience device including: an odor generation device including a cartridge that holds a tubular body including an odor component, a release unit that releases the odor component to the outside, and a placement drive unit that places a specific cartridge among a plurality of the cartridges in the vicinity of the release unit; and an information processing device that controls the placement drive unit.

### BRIEF DESCRIPTION OF DRAWINGS

Fig. 1 is a diagram illustrating a first embodiment of an odor generation device 1.
Fig. 2 is a diagram illustrating the first embodiment of the odor generation device 1.
Fig. 3A is a six-sided view illustrating an example of an embodiment of a cartridge 10.
Fig. 3B is a cross-sectional view taken along line P-P of the cartridge 10.
Fig. 3C is a cross-sectional view taken along line Q-Q of the cartridge 10.
Fig. 4 is a perspective view illustrating an example of an embodiment of a holding portion 11.
Fig. 5 is a perspective view illustrating an example of an embodiment of a movable portion 12.
Fig. 6 is a perspective view illustrating an example of an embodiment of a support unit 13.
Fig. 7 is a diagram schematically illustrating a state in which the cartridge 10 is held by the support unit 13 and viewed from above.
Fig. 8 is a perspective view illustrating an example of an embodiment of a cartridge unit 20.
Fig. 9 is a perspective view illustrating an example of the embodiment of the cartridge unit 20.
Fig. 10 is a diagram schematically illustrating an example of an embodiment different from Figs. 8 and 9 of the cartridge unit 20.
Fig. 11 is a diagram schematically illustrating an example of an embodiment different from Figs. 8 to 10 of the cartridge unit 20.
Fig. 12 is a perspective view illustrating a surface (back surface in Fig. 8 or 9) of an indwelling portion 202 without a plurality of the support units 13 in the cartridge unit 20.
Fig. 13 is a cross-sectional view illustrating an example of an embodiment of a guide unit 141.
Fig. 14 is a perspective view illustrating an example of an embodiment of a release unit 140.
Fig. 15 is a diagram for describing a state in which the release unit 140 and the guide unit 141 are engaged with each other.
Fig. 16 is a diagram for explaining the operation of a placement drive unit 152.
Fig. 17 is a diagram for explaining the operation of the placement drive unit 152.
Fig. 18 is a diagram for explaining an operation of releasing odor component-containing air K in the odor generation device 1.
Fig. 19 is a diagram illustrating a second embodiment of the odor generation device 1.
Fig. 20 is a diagram illustrating a second embodiment of the odor generation device 1.
Fig. 21 is a diagram for explaining a flow of air in the guide unit 141.
Fig. 22 is a diagram for explaining an operation of an exhaust unit.
Fig. 23 is a diagram for explaining an operation of an intake unit.
Fig. 24 is a diagram for explaining the operation of the intake unit.
Fig. 25 is a diagram illustrating a deodorizing operation in the odor generation device 1.
Fig. 26 is a diagram illustrating a third embodiment of the odor generation device 1.
Fig. 27 is a diagram illustrating the third embodiment of the odor generation device 1.
Fig. 28 is a diagram illustrating a fourth embodiment of the odor generation device 1.
Fig. 29 is a diagram for explaining an operation of the odor generation device 1 according to the fourth embodiment.
Fig. 30 is a diagram for explaining an operation different from that in Fig. 29 of the odor generation device 1 according to the fourth embodiment.
Fig. 31 is a diagram illustrating a fifth embodiment of the odor generation device 1.
Fig. 32 is a diagram illustrating an example of an embodiment of a guide unit 141 used in the odor generation device 1 according to the fifth embodiment.
Fig. 33 is a diagram illustrating an example of an embodiment of an olfactory examination or olfactory training system 3.
Fig. 34 is a diagram for explaining an operation of an olfactory examination or olfactory training in the olfactory examination or olfactory training system 3.
Fig. 35 is a diagram of the odor generation device 1 according to the fifth embodiment as viewed from a side of a front surface accommodation unit 14.
Fig. 36 is a diagram of the odor generation device 1 according to the fifth embodiment, which is different from Fig. 35, as viewed from the side of the front surface accommodation unit 14.
Fig. 37 is a cross-sectional view schematically illustrating a state in which outside air is guided.
Fig. 38 is a cross-sectional view schematically illustrating a state in which outside air is guided, different from Fig. 37.
Fig. 39 is a diagram of the odor generation device 1 according to the fifth embodiment, which is different from Figs. 35 and 36, as viewed from the side of the front surface accommodation unit 14.
Fig. 40 is a diagram for explaining an operation of the odor generation device 1 according to the fifth embodiment.
Fig. 41 is a perspective view of an odor generation device 1 according to a sixth embodiment.
Fig. 42 is a diagram illustrating an example of an embodiment of a holder 133.
Fig. 43 is a diagram illustrating an example of an embodiment of an intake flow path 1331.
Fig. 44 is a diagram illustrating an example of an embodiment of an area 1332 storing a deodorant 1330 and a flow path 1333 connecting the area 1332 and a ventilation path.
Fig. 45 is a partially enlarged view of Fig. 44.
Fig. 46 is a diagram illustrating a state in which the guide unit 141 is replaced.

### MODE FOR CARRYING OUT THE INVENTION

Hereinafter, preferred embodiments for carrying out the present technology will be described with reference to the drawings.

The embodiments to be described below are intended to illustrate examples of representative embodiments of the present technology, and the scope of the present technology will not be construed narrower by these embodiments. Note that the description will be made in the following order.
1. First embodiment (odor generation device 1)
   (1) Configuration example of odor generation device 1
      (1-1) Overall configuration
      (1-2) Cartridge 10
      (1-3) Holding portion 11
      (1-4) Movable portion 12
      (1-5) Support unit 13
      (1-6) Cartridge unit 20 for odor generation device
      (1-7) Front surface accommodation unit 14
      (1-8) Back surface accommodation unit 15
      (1-9) Placement drive unit 152
      (1-10) Position adjustment unit 16
   (2) Operation example of odor generation device 1
2. Second embodiment (odor generation device 1)
   (1) Configuration example of odor generation device 1
      (1-1) Overall configuration
      (1-2) Front surface accommodation unit 14
      (1-3) Position adjustment unit 16
      (1-4) Contact portion 17
      (1-5) Deodorization unit 18
      (1-6) Guide unit 141
      (1-7) Exhaust unit
      (1-8) Intake unit
   (2) Operation example of odor generation device 1
3. Third embodiment (odor generation device 1)
   (1) Configuration example of odor generation device 1
      (1-1) Overall configuration
      (1-2) Position adjustment unit 16
      (1-3) Guide unit 141
4. Fourth embodiment (odor generation device 1)
   (1) Configuration example of odor generation device 1
      (1-1) Overall configuration
      (1-2) Support unit 13 and cartridge unit 20 for odor generation device
      (1-3) Accommodation unit 40
   (2) Operation example of odor generation device 1
5. Fifth embodiment (odor generation device 1)
   (1) Configuration example of odor generation device 1
      (1-1) Overall configuration
      (1-2) Front surface accommodation unit 14 and back surface accommodation unit 15
      (1-3) Guide unit 141
      (1-4) Odor control unit 143
      (1-5) Airflow guidance portion 144
   (2) Operation example of odor generation device 1
6. Sixth embodiment (odor generation device 1)
   (1) Configuration example of odor generation device 1
      (1-1) Overall configuration
      (1-2) Holder 133
      (1-3) Guide unit 141
      (1-4) Drive mechanism unit 151
      (1-5) Bottom convex portion 161
7. Seventh embodiment (olfactory examination or olfactory training system 3)
   (1) Configuration example of olfactory examination or olfactory training system 3
      (1-1) Overall configuration
      (1-2) Information processing device 2
   (2) Operation example of olfactory examination or olfactory training system 3
8. Eighth embodiment (olfactory presentation neurodegenerative disease prevention and/or treatment device)
9. Ninth embodiment (odor experience device)

### 1. First embodiment (odor generation device 1)

### (1) Configuration example of odor generation device 1

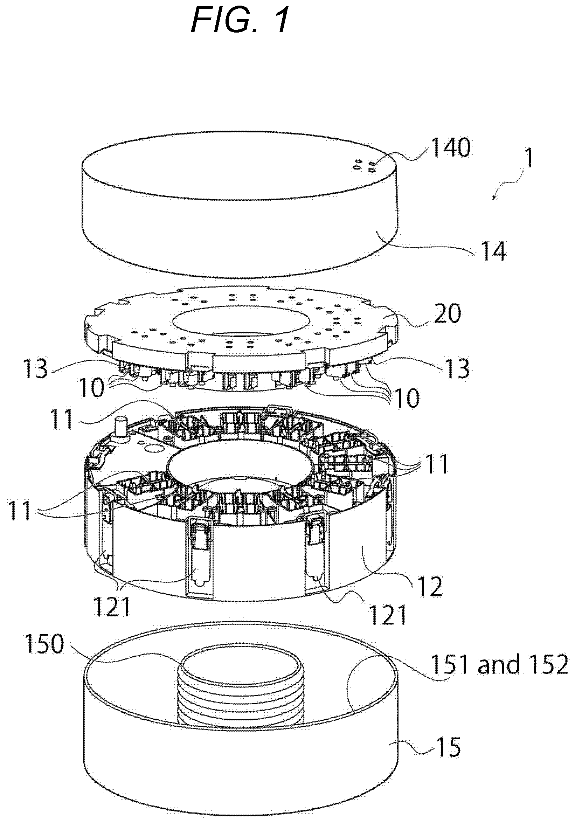

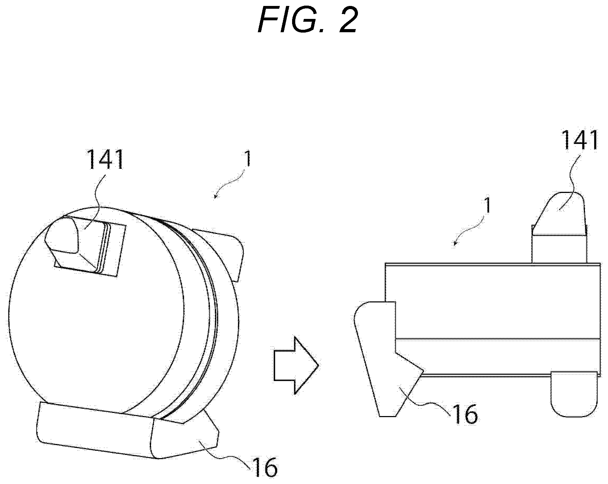

A configuration example of an odor generation device 1 according to the first embodiment of the present technology will be described with reference to Figs. 1 and 2. Fig. 2 illustrates an external appearance in which each part illustrated in Fig. 1 is engaged and a guide unit 141 and a position adjustment unit 16 are attached.

### (1-1) Overall configuration

The odor generation device 1 according to the present embodiment includes a cartridge 10, a holding portion 11, a movable portion 12, a support unit 13, a cartridge unit 20, a front surface accommodation unit 14 including a release unit 140, a guide unit 141, a back surface accommodation unit 15 including a drive mechanism unit 151 and a placement drive unit 152, and a position adjustment unit 16. Hereinafter, each part will be described in detail.

The odor generation device 1 is a device that releases an odor component in a tubular body held by a cartridge 10 described later to the outside. Note that, in the present technology, the "odor component" can include any component that stimulates a part or all of the receptors present in the nasal cavity, such as odor molecules. In the nasal cavity, besides olfactory receptors, receptors of the trigeminal nerve that control stimulation such as cold, hot, and pain exist, and the odor component in the present technology is a broad concept including all components that stimulate some or all of these receptors. Specifically, for example, in a case where menthol is used as an odor component, menthol can serve as a stimulus through an olfactory receptor as well as a cold stimulus through a receptor of the trigeminal nerve (TRPA1 channel).

The odor generation device 1 is used, for example, as a device that emits an odor to a space in a limited range, and is specifically used for an olfactory examination, olfactory training (olfactory stimulation therapy), and the like. Furthermore, the film may be mounted on an automobile, a head mounted display, a relaxed product such as a neck pillow or an eye pillow, or the like. Furthermore, the odor generation device 1 may be used as a device that emits odor to a wide range of space.

The odor generation device 1 may be a portable device that can be carried by a user or may be a stationary device.

### (1-2) Cartridge 10

The cartridge 10 holds a tubular body 102 containing the odor component. Examples of the tubular body 102 include a glass tube, a metal tube formed of stainless steel or the like, a ceramic tube, a ceramic tube, a ceramic tube, and the like, but the present technology is not limited thereto. Furthermore, examples of the structure of the tubular body 102 include a sealed structure, but the sealed structure is not necessarily required as long as an odor component can be contained, and a part thereof may be released.

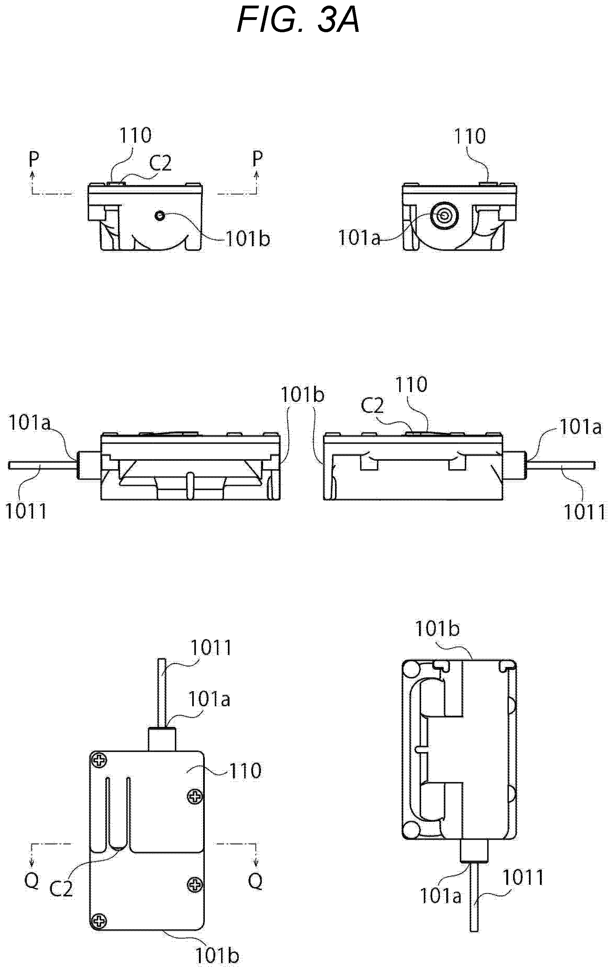

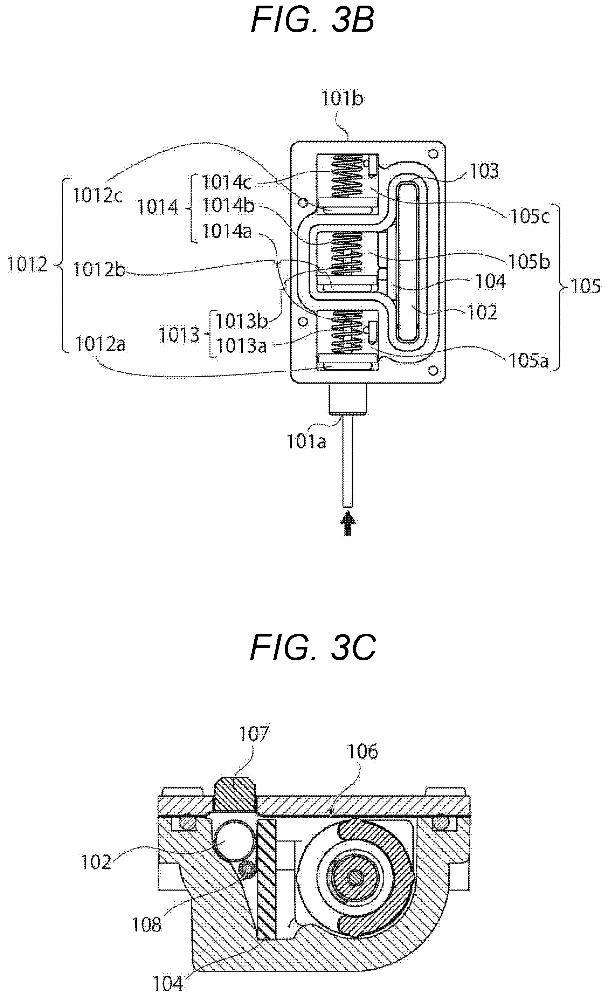

Fig. 3A is a six-sided view illustrating an example of an embodiment of the cartridge 10. Fig. 3B is a cross-sectional view of the cartridge 10 taken along line P-P. Fig. 3C is a cross-sectional view of the cartridge 10 taken along line Q-Q.

The cartridge 10 illustrated in Fig. 3 includes at least an opening 101 (101a, 101b), a tubular body 102, a tubular body holding portion 103, an odor holding body 104, a ventilation portion 105, and a crushing portion 106. Furthermore, a pressing portion 107, an odor component guidance portion 108, and the like may be provided as necessary.

In the cartridge 10, when the pressing portion 107 is pressed, a portion of the crushing portion 106 pressed by the pressing portion 107 is deformed in the direction of the tubular body 102, and pressure is applied to the tubular body 102 to crush the tubular body 102. The odor component is released from the crushed tubular body 102, and the released odor component is absorbed and held by the odor holding body 104.

In the cartridge 10, when one opening 101a (inflow opening 101a) is opened in a state where the odor component is held by the odor holding body 104, the air flows in from the outside. The flowed air flows into the ventilation portion 105 and mixes with the odor component held by the odor holding body 104. In this state, by opening the other opening 101b (release opening 101b), the odor component-containing air is released to the outside.

The ventilation portion 105 is divided into three sections (first ventilation portion 105a, second ventilation portion 105b, and third ventilation portion 105c), and two or three sealing lids 1012, two shafts 1013, and three springs 1014 are provided (first sealing lid 1012a, second sealing lid 1012b, third sealing lid 1012c, first shaft 1013a, second shaft 1013b, first spring 1014a, second spring 1014b, third spring 1014c), but the present technology is not limited thereto. Furthermore, the ventilation portion 105 may be a single section or two or more sections. Furthermore, the number of the sealing lid 1012, the shaft 1013, and the spring 1014 can also be freely designed according to the number of sections of the ventilation portion 105.

### (1-3) Holding portion 11

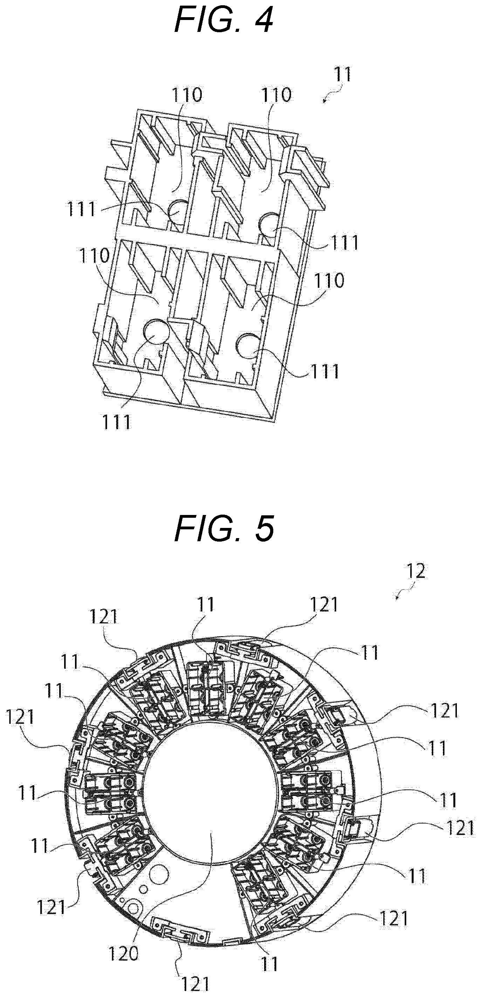

The holding portion 11 is a portion to which the plurality of cartridges 10 is attached and held. Furthermore, the holding portion 11 preferably includes a crushing mechanism that breaks the plurality of tubular bodies. The number of the cartridges 10 held by the holding portion 11 is four in the embodiment illustrated in Fig. 4, but is not limited thereto in the present technology.

As illustrated in Fig. 4, a space 110 to which the cartridge 10 can be attached is formed in the holding portion 11. The shape of the space 110 is not particularly limited, and can be adapted to the shape of the cartridge 10, and can be formed in, for example, a substantially rectangular parallelepiped shape, a substantially cylindrical shape, a substantially cubic shape, or the like. A communication hole 111 through which a pusher in the drive mechanism unit 151 to be described later communicates is formed in a lower surface of the holding portion 11.

The crushing mechanism includes both chemical means and physical means, and may be either contact or noncontact. Specifically, for example, there is a means in which the cartridge 10 includes a pressing portion 107 that transmits an external force to the tubular body 102, and the tubular body 102 is broken by pressing the pressing portion 107. In this case, the crushing mechanism includes a convex portion capable of pressing the pressing portion 107, and when the cartridge 10 including the pressing portion 107 is inserted into the holding portion 11, the pressing portion 107 and the convex portion come into contact with each other, and an external force is transmitted to the tubular body 102, so that the tubular body 102 is crushed.

A material forming the holding portion 11 is not particularly limited, and may be formed of, for example, an organic polymer material. As the organic polymer material, for example, one or more materials of polyvinyl chloride, polyethylene, a phenol resin, an olefin resin, nylon, polyester, synthetic rubber, a silicone resin, natural rubber, protein, nucleic acid, lipid, polysaccharide, and the like may be used. Furthermore, for example, one kind or two or more kinds of materials selected from polymer resins such as an acrylic resin, a urethane resin, an ABS resin, a polyether ether ketone (PEEK) resin, a polyacetal (POM) resin, a fluororesin, a cycloolefin polymer resin, and a polyimide resin, metals such as stainless steel and aluminum, inorganic crystals such as quartz, glass, and the like may be used. Moreover, the holding portion 11 may be formed to be porous, and as the porous material, for example, a mesh structure, cork, mesoporous silica, calcium carbonate, or the like can be used. Furthermore, besides the porous material, a fiber structure, a layered structure (for example, clay minerals and the like), ceramic, or the like can also be used.

### (1-4) Movable portion 12

The movable portion 12 is a portion including a plurality of holding portions 11. The number of holding portions 11 included in the movable portion 12 is 10 in the embodiment illustrated in Fig. 5, but is not limited thereto in the present technology.

The material forming the movable portion 12 is not particularly limited, and examples thereof include the materials similar to the examples of the material forming the holding portion 11, and thus the description thereof is omitted here.

The shape of the movable portion 12 is not particularly limited as long as it is formed so as to be able to hold the plurality of holding portions 11, and can be formed so as to conform to the shape of the holding portion 11.

The movable portion 12 preferably includes a plurality of holding portions 11 in parallel. Accordingly, space saving can be achieved. Examples of the form in which the movable portion 12 includes the plurality of holding portions 11 in parallel include a form in which the plurality of holding portions 11 are arranged in parallel in one direction, a form in which the plurality of holding portions 11 are arranged in parallel in one direction and a direction perpendicular to the one direction, and a form in which the plurality of holding portions 11 are arranged in parallel in an annular shape as illustrated in Fig. 5. In the embodiment illustrated in Fig. 5, a hole 120 is provided at the center of the movable portion 12 to be fitted to the back surface accommodation unit 15 to constitute a rotation mechanism. Note that the mode in which the movable portion 12 includes the plurality of holding portions 11 is preferably formed to be adapted to a mode in which the cartridge unit 20 described later supports the plurality of cartridges 10.

Furthermore, as illustrated in Fig. 5, the movable portion 12 preferably includes an engagement portion 121 that engages with the cartridge unit 20 including the plurality of holding portions 11. By having the engagement portion 121, a plurality of the tubular bodies 102 can be more reliably broken, and the force required to crush the plurality of tubular bodies 102 can be suppressed. The form of the engagement portion 121 is not particularly limited, and may be, for example, a threaded form (male screw or female screw), a locking tool such as a cam latch handle, a buckle structure, a lever structure used in a cutting machine or punching, an adhesive layer, or the like. In a case where the form of the engagement portion 121 is a form in which thread cutting processing is performed or the like, it is preferable that at least a part of the cartridge unit 20 has a portion to be engaged with the processing portion.

### (1-5) Support unit 13

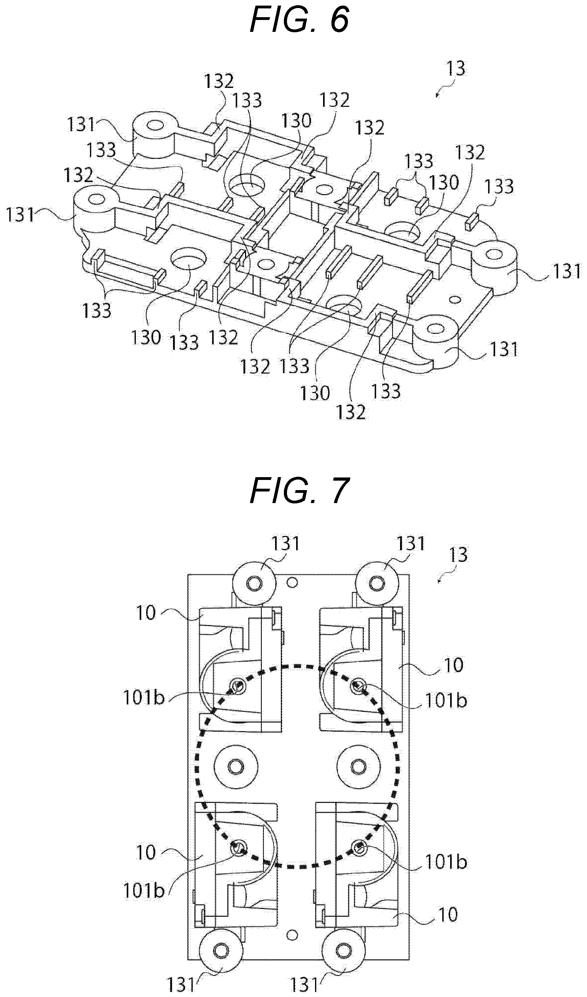

The support unit 13 is a portion that supports the plurality of cartridges 10. The number of the cartridges 10 supported by the support unit 13 is 4 in the embodiment illustrated in Fig. 6, but is not limited thereto in the present technology. However, in the present embodiment, since the support unit 13 is engaged with the holding portion 11 in a state of supporting the plurality of cartridges 10, the number of cartridges 10 supported by the support unit 13 and the number of cartridges 10 held by the holding portion 11 are preferably the same.

The material forming the support unit 13 is not particularly limited, and examples thereof include the materials similar to the examples of the material forming the holding portion 11, and thus the description thereof is omitted here.

The shape of the support unit 13 is not particularly limited as long as the support unit is formed to be able to support the plurality of cartridges 10, and the support unit can be formed to be adapted to the shape of the cartridge 10. In the present embodiment, a communication hole 130 for sending the taken air to the user is formed in the lower surface (surface on the side of the front surface accommodation unit 14) of the support unit 13, and a positioning mechanism 131 for fitting to the cartridge unit 20 is formed in at least a part of the support unit 13 as illustrated in Fig. 6.

Fig. 7 is a diagram schematically illustrating a state in which the cartridge 10 is held by the support unit 13 and viewed from above. The displacement of the plurality of cartridges 10 in the support unit 13 is not particularly limited, but as illustrated in Fig. 7, it is preferable to displace the plurality of cartridges 10 such that distances between the respective ejection openings 101b in the plurality of cartridges are uniform.

The method of engaging the plurality of cartridges 10 with the support unit 13 is not particularly limited, and examples thereof include a method of pushing each cartridge 10 into contact with the support unit 13 to attach the cartridge. In this case, the support unit 13 preferably includes a fixing portion 132 that fixes the movement of the cartridge 10 in the attaching and detaching direction. As a result, the cartridge 10 can be fixed at a desired position of the support unit 13 via the fixing portion 132. Note that, the embodiment illustrated in Fig. 6 shows a case where the form of the fixing portion 132 is a claw, but the form of the fixing portion 132 is not particularly limited.

In the present embodiment, one support unit 13 supports a plurality of the cartridges 10 having the same odor and having different concentrations of the odor. Specifically, in a case where the support unit 13 supports four cartridges 10, the concentration sequentially increases from the cartridge 10 at level 1 having the same odor and the lowest concentration of the odor in order of level 2 and level 3, and the cartridge 10 at level 4 having the highest concentration among the support unit 13 having the same concentration can be supported by one support unit 13.

### (1-6) Cartridge unit 20 for odor generation device

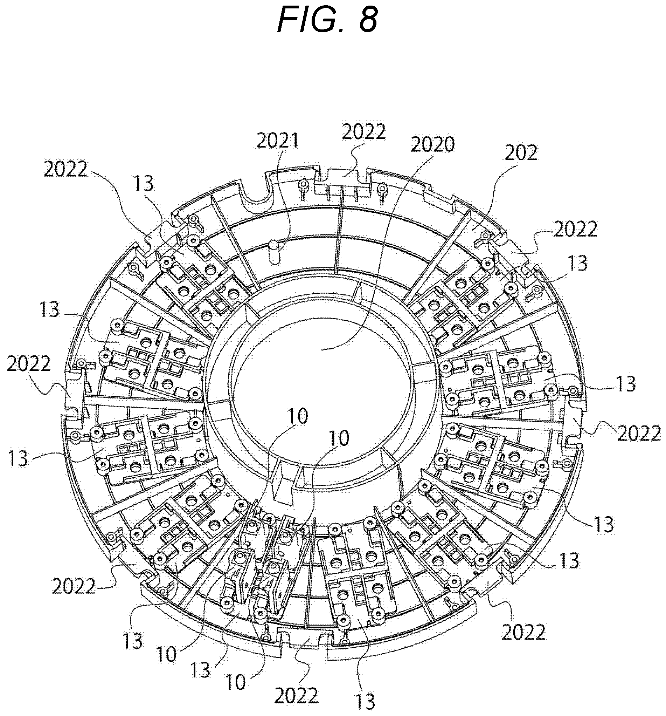

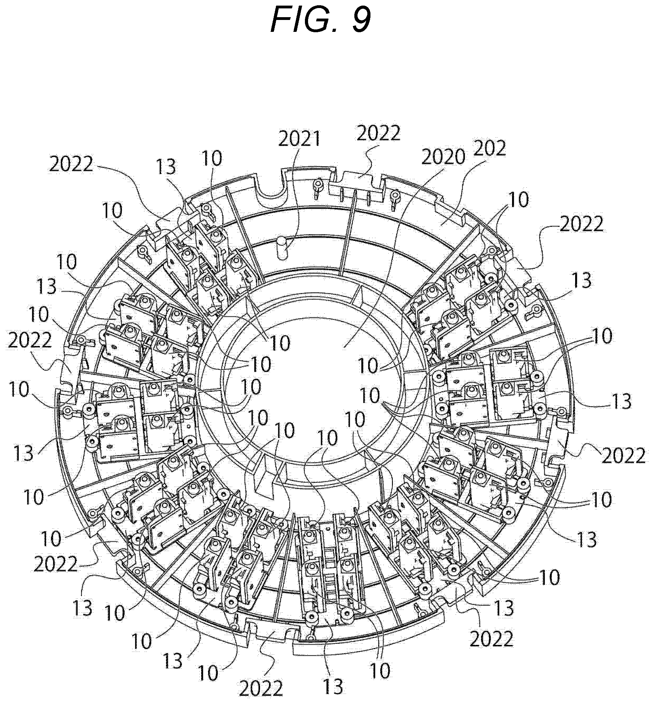

The cartridge unit 20 for an odor generation device (also simply referred to as "cartridge unit 20") is used in the odor generation device 1 and includes a plurality of cartridges 10 and a plurality of support unit 13. The number of the support unit 13 included in the cartridge unit 20 is, for example, 10 in the embodiment illustrated in Figs. 8 and 9, but is not limited thereto in the present technology. However, in the present embodiment, since the cartridge unit 20 is engaged with the movable portion 12 in a state of having the plurality of support units 13, the number of the holding portions 11 held by the movable portion 12 and the number of the support units 13 of the cartridge unit 20 are preferably the same.

The form of the cartridge unit 20 is not particularly limited as long as it is formed so as to include the plurality of cartridges 10 and the plurality of support units 13, and can be formed so as to be adapted to the shapes of the cartridge 10 and the support units 13. Note that Fig. 8 schematically illustrates a mode in which the cartridge unit 20 includes the plurality of cartridges 10 only in some of the support units 13, and Fig. 9 schematically illustrates a mode in which the cartridge unit 20 includes the plurality of cartridges 10 in all of the support units 13, but the present technology may include any mode.

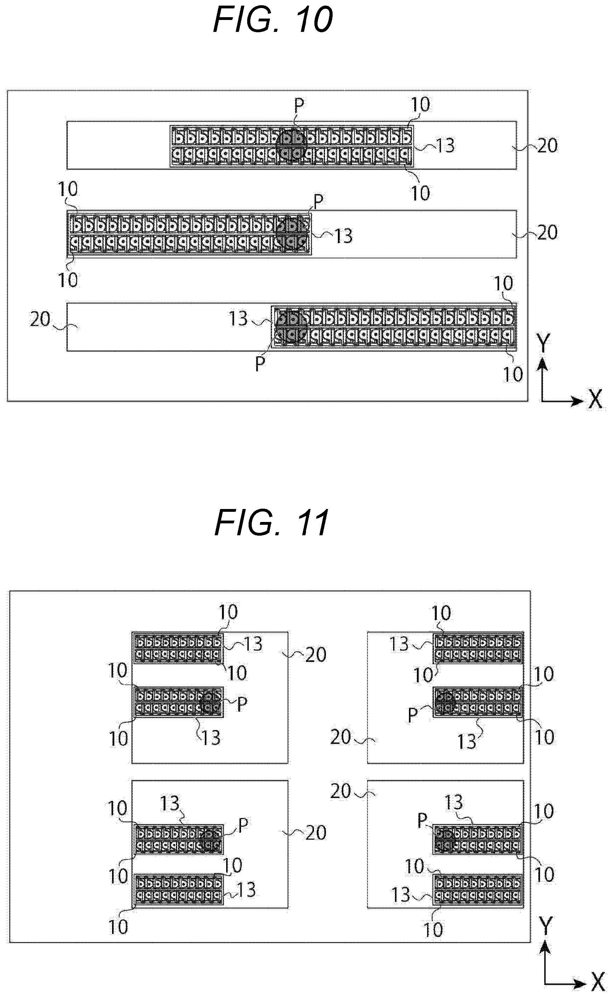

Fig. 10 is a diagram schematically illustrating an example of an embodiment of the cartridge unit 20 different from Figs. 8 and 9.

In the embodiment illustrated in Fig. 10, a case where a plurality of cartridges 10 is supported in parallel in one direction (X direction in Fig. 10) is illustrated. In the present embodiment, the cartridge 10 (portion P in Fig. 10) that discharges the odor can be changed by moving the support unit 13 and the cartridge unit 20 in the X direction in Fig. 10. Note that, in the embodiment illustrated in Fig. 10, the cartridge unit 20 may not only support the plurality of cartridges 10 in one plane but also support the plurality of cartridges 10 in parallel in the Z-axis direction (not illustrated).

Fig. 11 is a diagram schematically illustrating an example of an embodiment different from Figs. 8 to 10 of the cartridge unit 20.

In the embodiment illustrated in Fig. 11, a case where a plurality of cartridges 10 is supported in parallel in one direction and a direction perpendicular to the one direction (XY direction in Fig. 11) is illustrated. In a case where the number of cartridges 10 is large, the present embodiment can achieve space saving as compared with the embodiment illustrated in Fig. 10. In the present embodiment, by moving the support unit 13 or the cartridge unit 20 in the X direction or the Y direction in Fig. 11, the cartridge 10 (portion P in Fig. 11) that discharges the odor can be changed. Note that, in the embodiment illustrated in Fig. 11, the cartridge unit 20 may not only support the plurality of cartridges 10 in one plane but also support the plurality of cartridges 10 in parallel in the Z-axis direction (not illustrated).

In the embodiment illustrated in Figs. 8 and 9, a case where the plurality of cartridges 10 is supported in an annular shape is illustrated. In the present embodiment, since the entire cartridge unit 20 is rotationally driven, the odor component can be transferred to the odor holding body 104 using the centrifugal force when the odor component is transferred to the odor holding body 104 in the cartridge 10, so that the odor can be efficiently generated. Note that the cartridge unit 20 may support not only the plurality of cartridges 10 in one plane but also the plurality of cartridges 10 in a plurality of planes in an annular shape.

In the present embodiment, the cartridge unit 20 preferably includes the indwelling portion 202 in which the plurality of support units 13 is indwelled as in the embodiment illustrated in Figs. 8 and 9. The form of the indwelling portion 202 is not particularly limited, and can be formed in conformity with the support unit 13 and the displacement form thereof. For example, the support unit 13 can be indwelled by being fitted with one or a plurality of the positioning mechanisms 131 formed in the support unit 13. In the embodiment illustrated in Figs. 8 and 9, the indwelling portion 202 supports the plurality of cartridges 10 in a multiple annular shape in plan view, and in a case where the number of cartridges 10 is large, the plurality of cartridges 10 is supported in a multiple annular shape in plan view, so that more cartridges 10 can be supported and space saving can be achieved.

The material forming the indwelling portion 202 is not particularly limited, and for example, one or more materials of organic polymer materials, acrylic resins, urethane resins, ABS resins, polyether ether ketone (PEEK) resins, polyacetal (POM) resins, fluororesins, cycloolefin polymer resins, polymer resins such as polyimide resins, metals such as stainless steel and aluminum, and the like may be used.

In this case, for example, the indwelling portion 202 may be formed in a substantially circular shape as illustrated in Figs. 8 and 9, and may have a hole 2020 at the center thereof as illustrated in Figs. 8 and 9, and the hole 2020 can be used as a positioning mechanism when engaging with the movable portion 12. Thus, for example, the rotation mechanism can be configured in a state of being engaged with the movable portion 12. Furthermore, a protrusion 2021 or the like may be provided as another member constituting the positioning mechanism. Note that the hole 2020 may be a hole instead of a through hole.

Furthermore, as illustrated in Figs. 8 and 9, the indwelling portion 202 preferably includes a positioning mechanism 2022 for engaging with the movable portion 12 particularly at the peripheral edge. As a result, the cartridge unit 20 can be engaged with the movable portion 12 at a desired position. Furthermore, in a case where the engagement portion 121 is a fixing tool having a buckle structure, the buckle portion can be configured to be locked to the positioning mechanism 2022.

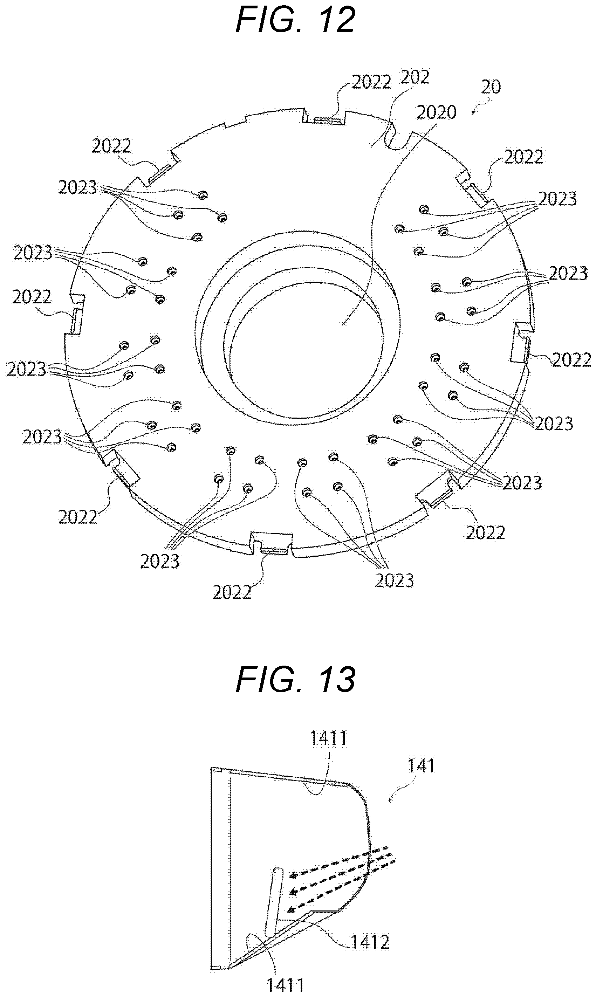

Fig. 12 is a diagram schematically illustrating a surface (back surface in Figs. 8 and 9) of the indwelling portion 202 not having the plurality of support units 13 in the embodiment illustrated in Figs. 8 and 9. The indwelling portion 202 preferably includes a discharge hole 2023 for discharging the odor component. The number of the discharge holes 2023 is more preferably plural, and particularly preferably the same as the number of the cartridges 10 supported by the cartridge unit 20. Furthermore, the discharge hole 2023 is preferably located at a position corresponding to the communication hole 130 of the support unit 13. In this case, the airflow sent from the communication hole 130 can be discharged through the discharge hole 2023.

In the present embodiment, in the cartridge unit 20, in a case where a plurality of the cartridges 10 having the same odor and having different concentrations of the odor are supported by the first support unit 13a, a plurality of the cartridges 10 having the same odor and having a concentration different from the concentration of the plurality of the cartridges 10 supported by the first support unit 13a may be supported by the second support unit 13b. Specifically, in a case where the first support unit 13a supports the four cartridges 10, the concentration increases in order of level 2 and level 3 from the cartridge 10 at level 1 having the same odor and the lowest concentration of the odor, and in a case where the cartridge 10 at level 4 having the highest concentration in the first support unit 13a is supported by the first support unit 13a, and the second support unit 13b also supports the four cartridges 10, the concentration increases in order of level 6 and level 7 from the cartridge 10 at level 5 having a concentration higher than the highest concentration in the first support unit 13a, and the cartridge 10 at level 8 having the highest concentration in the second support unit 13b can be supported by the second support unit 13b. In this case, the cartridge unit 20 may include a plurality of types (for example, five types) of odors, and may include the cartridge 10 that holds odors at a plurality of different concentrations (for example, eight concentrations of Level 1 to Level 8) for each odor.

Note that, in the cartridge unit 20, as illustrated in Figs. 11 and 12, a plurality of cartridges 10 may be attached in advance to some or all of the support unit 13. In this case, the cartridge unit 20 may be distributed in a state where the plurality of cartridges 10 is provided in advance to some or all of the support unit 13.

Furthermore, in the present embodiment, in a case where there is no odor in a part or all of the cartridges 10, in a case where a certain period has elapsed since the cartridge unit 20 was purchased, or the like, the user can cope with such a case by replacing only the cartridge unit 20. Therefore, the cartridge unit 20 according to the present technology can be distributed alone. Furthermore, it is also possible to replace only some or all of the cartridges 10 in the cartridge unit 20. Accordingly, the cartridge 10 can also be distributed alone.

Moreover, although not illustrated, the odor generation device 1 may have a disposal mechanism that discards a part or all of the cartridges 10 having no odor and the cartridge unit 20 in a case where the odor in a part or all of the cartridges 10 has disappeared or in a case where a certain period has elapsed since the cartridge unit 20 was purchased.

### (1-7) Front surface accommodation unit 14

As illustrated in Fig. 1, the front surface accommodation unit 14 is provided with a release unit 140 capable of communicating with the discharge hole 2023 of the cartridge unit 20. Odor component-containing air is released to the outside through the release unit 140.

In the present embodiment, the release unit 140 includes a guide unit 141 that guides the airflow containing the odor component in the vicinity of the nose of the user as illustrated in Fig. 2. The material forming the guide unit 141 is not particularly limited, and examples thereof include paper (including recycled paper), wood, bamboo sheath, plastic, and coal. A part or all of the guide unit 141 may be detachably formed, and in this case, for example, may be disposable for each user.

Furthermore, the guide unit 141 may include an odor component capturing portion 1411. In the embodiment illustrated in Fig. 13, the odor component capturing portion 1411 is formed so as to be attached to the back surface of the guide unit 141. The odor component capturing portion 1411 is, for example, a deodorant or an adsorbent, and may be one in which a gas adsorbing substance such as activated carbon is woven, kneaded, or a fiber or a structure itself has a gas adsorption structure, or may have a filter structure that always passes until an air flow is discharged.

The material forming the odor component capturing portion 1411 is not particularly limited, and examples thereof include activated carbon (for example, palm, fir, bamboo, resin, wood, fiber, coal, and the like), zeolite, a layered compound (for example, sheet silicate, alumina, and the like), and porous silica (including mesoporous silica). Furthermore, the shape of the odor component capturing portion 1411 is not particularly limited, and may be a powder, a granular shape, a fibrous shape, a molded article, a sheet shape (for example, a nonwoven fabric, a woven fabric, or the like), a rod shape, a plate shape, or the like. Furthermore, the surface treatment of the odor component capturing portion 1411 does not matter whether or not the activation treatment is performed, and does not matter whether or not the surface functional group exposure treatment (for example, acidification, alkalization, polarisation, non-polarisation, etc.) is performed.

The guide unit 141 may include an inflow prevention portion 1412 that prevents the inflow of exhalation from the nose and/or the mouth of the user. In the embodiment illustrated in Fig. 13, a plate-shaped inflow prevention portion 1412 is provided in the guide unit 141 in order to prevent spray generated by, for example, a user's sneezing. The material forming the inflow prevention portion 1412 is not particularly limited, and examples thereof include paper (including recycled paper), wood, bamboo sheath, plastic, coal, and the like. Similarly to the guide unit 141 described above, a part or all of the inflow prevention portion 1412 may be detachably formed, and in this case, for example, may be disposable for each user.

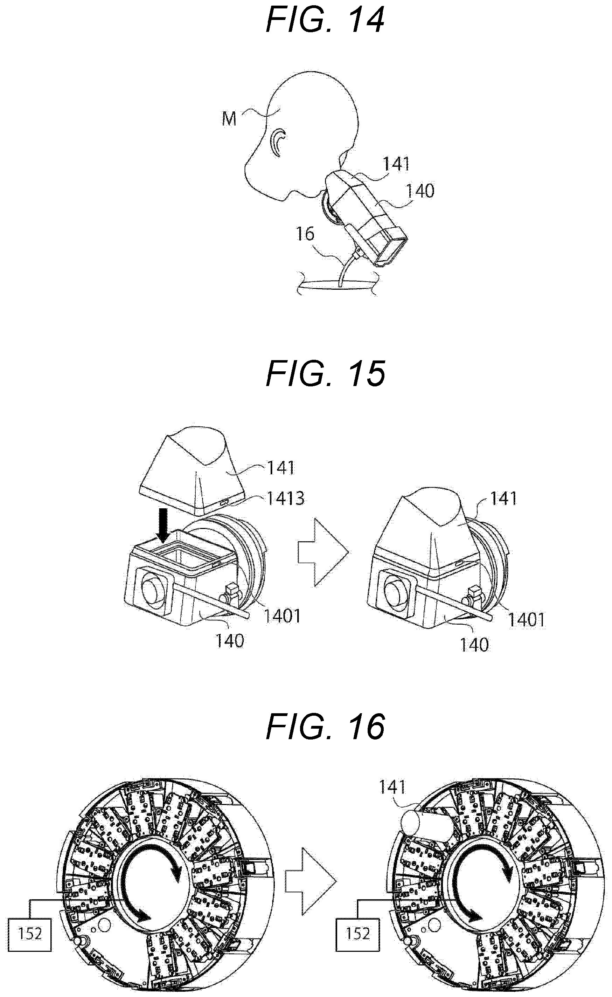

Fig. 14 is a diagram schematically illustrating a modification of the release unit 140. The release unit 140 may be formed to protrude from a housing as illustrated in Fig. 14.

Fig. 15 is a diagram for describing a state in which the release unit 140 and the guide unit 141 are engaged with each other.

As illustrated in Fig. 15, the guide unit 141 may have an engagement portion 1413 that engages with a part of the release unit 140 or a part of the front surface accommodation unit 14. In this case, it is preferable that an engaged portion 1401 to be engaged with the engagement portion 1413 is provided in a part of the release unit 140 or a part of the front surface accommodation unit 14. In the embodiment illustrated in Fig. 15, for example, a case where the engagement portion 1413 has a concave shape and the engaged portion 1401 has a convex shape is illustrated, but these shapes may be reversed, that is, the engagement portion 1413 may have a convex shape and the engaged portion 1401 may have a concave shape. Furthermore, the form of the engagement portion 1413 may be not only the above-described uneven shape but also a form (male screw or female screw) subjected to thread cutting processing, a locking tool such as a cam latch handle, a buckle structure, a lever structure used in a cutting machine or hole punching, an adhesive layer, and the like.

### (1-8) Back surface accommodation unit 15

The back surface accommodation unit 15 is provided with a fitting portion 150 to be fitted with the movable portion 12. The fitting portion 150 is preferably configured to be able to withstand a load applied in the attaching and detaching direction of the cartridge 10 when the plurality of cartridges 10 is crushed.

Furthermore, the back surface accommodation unit 15 includes a drive mechanism unit 151. Moreover, a substrate for energizing the drive mechanism unit 151, the placement drive unit 152, and the like on the basis of an instruction or the like from a user interface unit 21 may be provided. The drive mechanism unit 151 includes a drive mechanism accommodating portion, and is connected to the operation shaft and the first shaft 1013a in the cartridge 10 to drive them. The drive mechanism accommodating portion can have, for example, a cylindrical shape, but can be adapted to the shape of the cartridge 10 and formed in a cylindrical shape, a rectangular parallelepiped shape, a cubic shape, or the like.

The drive mechanism unit 151 includes a pusher connected to the operation shaft and a shape memory alloy SMA of a thin wire which is a drive source for driving the pusher inside the drive mechanism accommodating portion. A rear end of the pusher is fixed to a drive mechanism fixing portion provided at the inner rear end of the drive mechanism accommodation portion. An SMA sliding portion for folding back and sliding the shape memory alloy SMA is provided near the distal end of the pusher. Furthermore, the entire drive mechanism unit 151 is fixed by a support or the like attached below the drive mechanism accommodating portion, and a wiring capable of supplying power is connected to a rear end of the shape memory alloy SMA located in the drive mechanism fixing portion.

The pusher is movable inside the drive mechanism accommodation portion in the extending direction by expansion and contraction of the shape memory alloy SMA. The shape of the pusher is not particularly limited as long as it is a shape that pushes the operation axis, and may be a cylindrical shape, a conical shape, a cylindrical shape, a prismatic shape, or the like.

The shape memory alloy SMA is folded back in a U-shape at the SMA sliding portion provided near the distal end of the pusher and passes through the inside of the pusher, and both ends thereof are fixed to the drive mechanism fixing portion located at the rear end of the pusher. Moreover, the actuator as the drive source is not limited to the shape memory alloy SMA, and may be, for example, a linear motion mechanism that linearly moves a pusher such as a motor, a solenoid, a linear slide type, a pneumatic (air pump type), or a small electromagnet. Here, the linear motion mechanism includes not only a case where one member moves in the linear direction but also a case where a part of a plurality of members connected to each other moves in the linear direction.

### (1-9) Placement drive unit 152

As illustrated in Fig. 16, the placement drive unit 152 is a unit where a specific cartridge 10 among the plurality of cartridges 10 is placed in the vicinity of the release unit 140. Since the odor generation device 1 includes the placement drive unit 152, any cartridge 10 can be moved to the vicinity of the release unit 140, and the odor component held by the odor holding body 104 of the cartridge 10 can be delivered to a desired position (for example, the tip of the nose of the user, or the like). Furthermore, as a result, it is possible to suppress as much as possible the odor from adhering to the members constituting the odor generation device 1. In the present embodiment, the placement drive unit 152 is provided in the above-described back surface accommodation unit 15. The back surface accommodation unit 15 includes a substrate or the like for energizing the placement drive unit 152 based on an instruction or the like from the user interface unit 21.

The placement drive unit 152 can be driven in conformity with the forms of the support unit 13, the cartridge unit 20, and the like described above. For example, in a case where the cartridge unit 20 is in the embodiment illustrated in Fig. 10, the placement drive unit can be linearly driven. In a case where the cartridge unit 20 is in the form illustrated in Fig. 11, the placement drive unit can be XY-axis driven. In a case where the cartridge unit 20 is in the forms illustrated in Figs. 8 and 9, the placement drive unit can be rotationally driven as illustrated in Fig. 16.

The placement drive unit 152 is not particularly limited, and a conventionally known actuator or the like can be used.

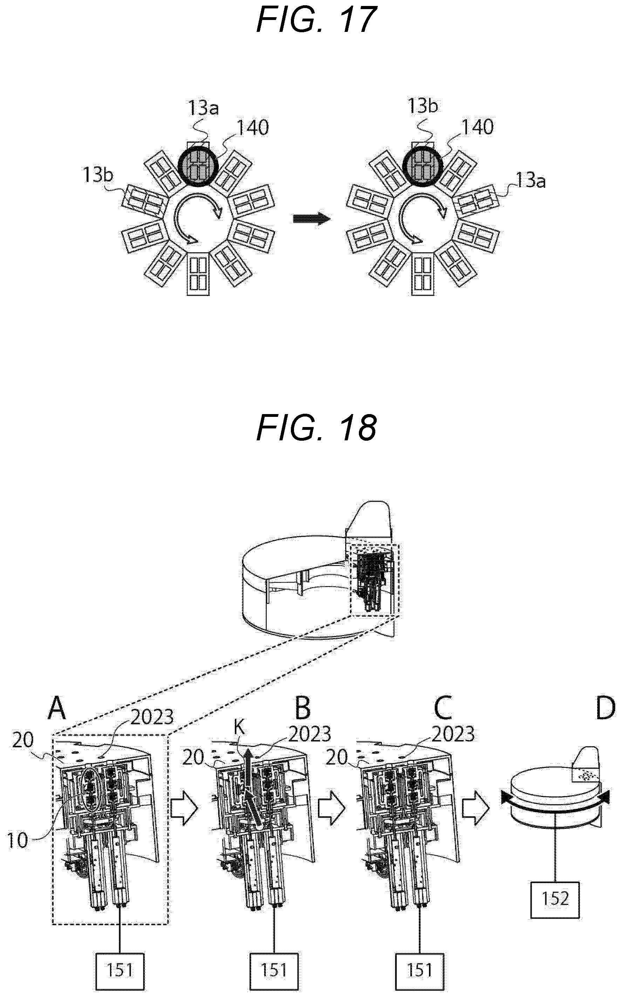

In a case where the support unit 13 supports a plurality of the cartridges 10 having the same odor and having different concentrations of the odor, the placement drive unit 152 preferably rotationally drives each support unit 13 as illustrated in Fig. 17. As a result, an odor can be provided to the user for each of the same odors.

Furthermore, in this case, as illustrated in Fig. 17, the placement drive unit 152 preferably moves the second support unit 13b that supports the plurality of cartridges 10 having the same odor and having a concentration of the odor different from the concentration of the plurality of cartridges 10 supported by the first support unit 13a, after the first support unit 13a that supports the plurality of cartridges 10 having the same odor and having different concentrations of the odor. Specifically, in a case where the first support unit 13a and the second support unit 13b support the four cartridges 10 as described above, it is possible to provide the user with an odor having the same odor and a concentration of levels 1 to 4, and then move the second support unit 13b to provide the user with an odor having the same odor and a concentration of levels 5 to 8. In this case, the cartridge unit 20 may include a plurality of types (for example, five types) of odors, and odors having a concentration of levels 1 to 8 may be provided to the user in a desired order for each odor.

### (1-10) Position adjustment unit 16

The position adjustment unit 16 is a portion capable of adjusting the position of the guide unit 141 described above.

The form of the position adjustment unit 16 is not particularly limited as long as the position of the guide unit 141 can be adjusted, but for example, as illustrated in Fig. 2, by providing a stand capable of fixing the position of the guide unit 141 on the side surface of the housing or the like, the user can freely adjust the height and angle of the guide unit 141, and it is possible to eliminate the positional deviation caused by the sex, the height of the chair, the height of the desk, or the like, and to sniff in a comfortable posture. Furthermore, as illustrated in Fig. 14, in a case where the release unit 140 is formed to protrude from the housing, a stand that can be freely bent and fixed in position may be provided on the lower surface of the release unit 140. Furthermore, examples of other forms include a jack (including mechanical, liquid-actuated, and air-actuated), a stand supported by a rod, a platelike member, a shape memory material, or the like, a flip mechanism, and the like. Furthermore, as the position adjustment unit 16, the number of jacks, stands, and the like may be plural.

Furthermore, the position adjustment unit 16 can adjust the position of the housing such that the indwelling portion 202 of the cartridge unit 20 is substantially horizontal to the installation surface when the cartridge unit 20 is replaced. As a result, the cartridge unit 20 can be easily replaced, and usability is improved. Furthermore, in a case where the cartridge unit 20 is engaged with the movable portion 12 to break the plurality of tubular bodies 102, the external force is easily applied by adjusting the position of the housing.

### (2) Operation example of odor generation device 1

An odor generating operation example of the odor generation device 1 according to the present embodiment will be described below with reference to Fig. 18.

First, the placement drive unit 152 that has received an instruction via an information processing device 2 or the like to be described later energizes the actuator to rotate together with the entire cartridge unit 20, and places the support unit 13 that supports the cartridge 10 having a specific odor in the vicinity of the release unit 140.

Next, as illustrated in Fig. 18A, the drive mechanism unit 151 that has received the instruction via the information processing device 2 described later or the like contracts the shape memory alloy SMA while sliding the shape memory alloy SMA through the SMA sliding portion by energization from the wiring to the shape memory alloy SMA as a drive source. Next, when the shape memory alloy SMA is shrunk, the drive mechanism fixing portion connected to the end portion of the shape memory alloy SMA moves the pusher in the direction of the cartridge 10.

When the pusher moves in the direction of the cartridge 10, the operation shaft connected to the tip of the pusher pushes the first sealing lid 1012a toward the inside of the first ventilation portion 105a, the inflow opening 101a is opened, and air flows into the first ventilation portion 105a. At this time, the first spring 1014a is contracted by the first sealing lid 1012a.

When the first sealing lid 1012a is pushed toward the inside of the first ventilation portion 105a, the first shaft 1013a attached to the first sealing lid 1012a moves in the second ventilation portion 105b direction. The first shaft 1013a pushes the second sealing lid 1012b toward the inside of the second ventilation portion 105b, and the air in the first ventilation portion 105a flows into the second ventilation portion 105b. At this time, the second spring 1014b is contracted by the second sealing lid 1012b.

The air flowing into the second ventilation portion 105b mixes with the odor component held by the odor holding body 104 to be described later to generate the odor component-containing air K.

When the second sealing lid 1012b is pushed toward the inside of the second ventilation portion 105b, the second shaft 1013b attached to the second sealing lid 1012b moves in the third ventilation portion 105c direction. The second shaft 1013b pushes the third sealing lid 1012c toward the inside of the third ventilation portion 105c, and the odor component-containing air K of the second ventilation portion 105b flows into the third ventilation portion 105c. At this time, the third spring 1014c is contracted by the third sealing lid 1012c.

Since the third ventilation portion 105c communicates with the release opening 101b, the odor component-containing air K flowing into the third ventilation portion 105c is discharged from the release opening 101b. Then, as illustrated in Fig. 18B, the odor component-containing air K flows through the release opening 101b and is released from the release unit 140 of the front surface accommodation unit 14 via the communication hole 130 of the support unit 13 and the discharge hole 2023 of the cartridge unit 20. The released odor component-containing air K is guided to the vicinity of the nose of the user by the guide unit 141.

Next, as illustrated in Fig. 18C, when the actuator is turned off after the odor component-containing air is released to the outside, the shrunk state of the shape memory alloy SMA in the non-energized state is released, and the pusher returns to the original position, so that the first sealing lid 1012a is also returned to the original position by the restoring force of the shrunk first spring 1014a. Furthermore, the second sealing lid 1012b is returned to the original position by the restoring force of the second spring 1014b, and the third sealing lid 1012c is returned to the original position by the restoring force of the third spring 1014c.

The first ventilation portion 105a and the second ventilation portion 105b can be sealed by the sliding capping mechanism using such first sealing lid 1012a, second sealing lid 1012b, and third sealing lid 1012c.

Note that the drive mechanism unit 151 is a mechanism that does not apply a load to the shape memory alloy SMA in the non-energized state. Furthermore, in the present embodiment, the shape memory alloy SMA is used as an example, but the present invention is not limited thereto, and an elastic body or the like capable of moving the pusher in the front-rear direction may be used.

Then, as illustrated in Fig. 18D, the placement drive unit 152 that has received an instruction via the information processing device 2 described later or the like rotates and drives the entire cartridge unit 20 by energizing the actuator, and places the support unit 13 that supports the cartridge 10 having the next specific odor in the vicinity of the release unit 140. Thereafter, the operation returns to the operation illustrated in Fig. 18A.

### 2. Second embodiment (odor generation device 1)

### (1) Configuration example of odor generation device 1

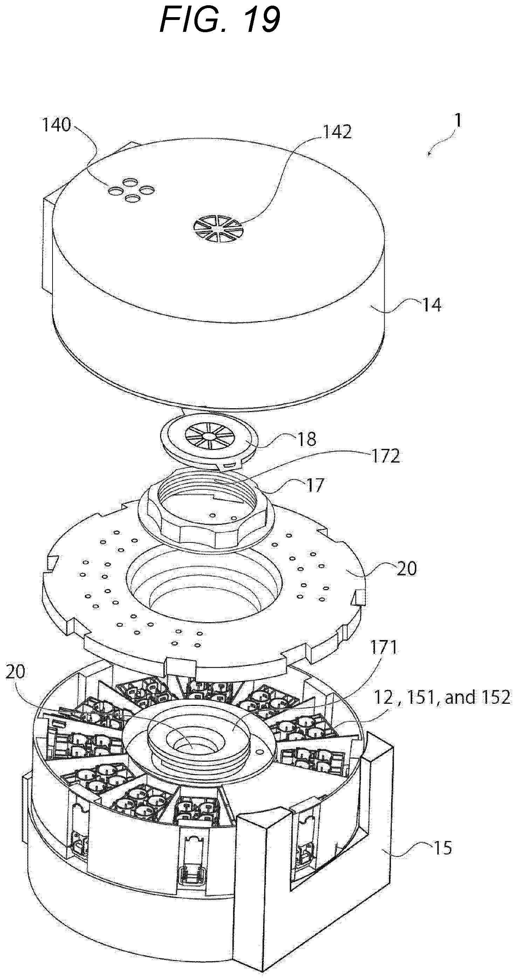

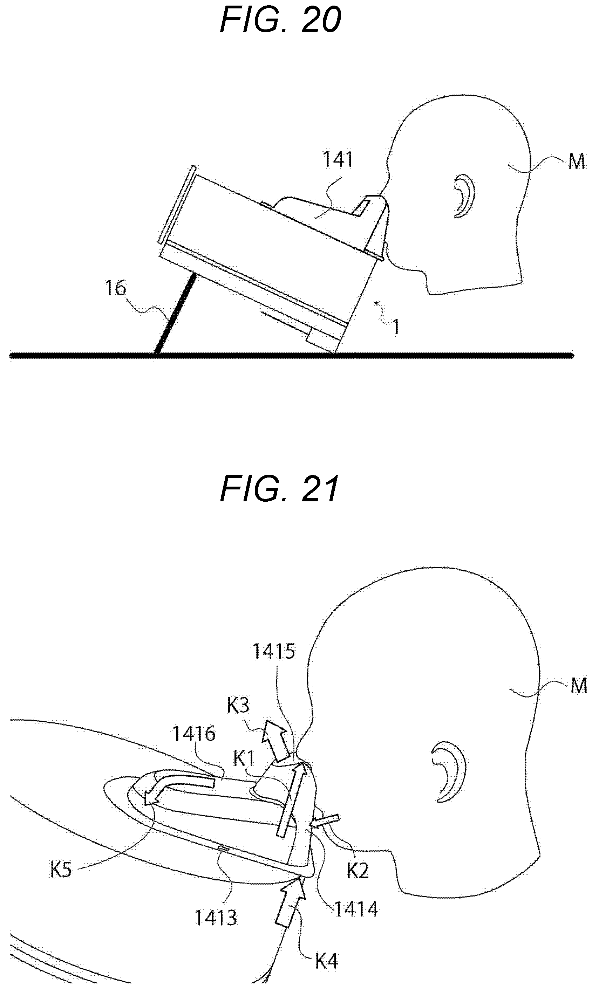

A configuration example of the odor generation device 1 according to the first embodiment of the present technology will be described with reference to Figs. 19 and 21. The present embodiment is a modification of the odor generation device 1 according to the first embodiment and the second embodiment described above.

### (1-1) Overall configuration

The odor generation device 1 according to the present embodiment includes the cartridge 10, the holding portion 11, the movable portion 12, the support unit 13, the cartridge unit 20, the front surface accommodation unit 14 including the release unit 140 and the communication hole 142, the guide unit 141, the back surface accommodation unit 15 including the drive mechanism unit 151 and the placement drive unit 152, the position adjustment unit 16, the contact portion 17, the deodorization unit 18, the exhaust unit, the intake unit, and the duct 19. Note that Fig. 20 illustrates a state in which the movable portion 12 having the plurality of holding portions 11, the drive mechanism unit 151, the placement drive unit 152, the exhaust unit, and the intake unit are already engaged with the back surface accommodation unit 15 in order from the upper surface side (side of the front surface accommodation unit 14). Hereinafter, each part will be described in detail.

Note that, in the present embodiment, the cartridge 10, the holding portion 11, the movable portion 12, the support unit 13, the cartridge unit 20, and the placement drive unit 152 are similar to those described above, and thus description thereof is omitted here.

### (1-2) Front surface accommodation unit 14

The front surface accommodation unit 14 includes a communication hole 142 communicating with a deodorization unit 18 to be described later in addition to the release unit 140 communicable with the discharge hole 2023 of the cartridge unit 20. As a result, the remaining odor component-containing air can be guided to the deodorization unit 18. The form of the communication hole 142 is not particularly limited, and can be formed to be adapted to the form of the deodorization unit 18 and the like.

### (1-3) Position adjustment unit 16

In the present embodiment, as illustrated in Fig. 20, the position adjustment unit 16 is provided with the position adjustment unit 16 capable of fixing the position of the guide unit 141 on the bottom surface of the substantially cylindrical housing, so that the user can freely adjust the height and angle of the guide unit 141 and can smell the odor in a comfortable posture. Note that, also in the present embodiment, the position adjustment unit 16 can adjust the position of the housing such that the indwelling portion 202 of the cartridge unit 20 is horizontal at the time of replacement of the cartridge unit 20.

### (1-4) Contact portion 17

The contact portion 17 is a portion that is screwed into a housing including the movable portion 12 and the cartridge unit 20 and comes into contact with the housing.

The form of the contact portion 17 is not particularly limited, and for example, in a case where the contact portion 17 is a lead screw, a screw groove 172 of the lead screw is pressed against and screwed to a screw protrusion 171 formed at the center of a housing including the movable portion 12 and the cartridge unit 20 using an external force applied by a drive motor or a user as a power source.

The material forming the contact portion 17 is also not particularly limited, and for example, one or two or more materials of organic polymer materials, polymer resins such as acrylic resins, urethane resins, ABS resins, polyether ether ketone (PEEK) resins, polyacetal (POM) resins, fluororesins, cycloolefin polymer resins and polyimide resins, metals such as stainless steel and aluminum, and the like may be used.

### (1-5) Deodorization unit 18

The deodorization unit 18 is a portion that deodorizes the remaining odor component-containing air retained in the guide unit 141.

For example, as illustrated in Fig. 19, the deodorization unit 18 is disposed at the bottom of the front surface accommodation unit 14. The deodorization unit 18 can operate in the vertical direction, the horizontal direction, or the rotation direction, and the power source of the deodorization unit 18 may be an actuator or an air flow.

The form of the deodorization unit 18 is not particularly limited, but may be a filter structure. In this case, for example, a slit structure in which a gas adsorbing substance such as activated carbon is woven or kneaded, or a fiber or a structure itself has a gas adsorbing structure can be applied to the filter. Furthermore, the slit structure may be a linear slit, a honeycomb structure, a structure in which a substantially circular shape, a substantially square shape, or the like is divided by a plurality of straight lines such that each area is equally spaced, or the like.

### (1-6) Guide unit 141

Fig. 21 is a diagram for explaining the flow of air in the guide unit 141. The guide unit 141 includes a nose cover portion 1414 that covers the nose of the user, an opening 1415 provided on the upper end side of the nose cover portion 1414, and a guidance portion 1416 that guides the remaining odor component-containing air to the deodorization unit 18. Furthermore, in the embodiment illustrated in Fig. 21, the guide unit 141 is engaged with a part of the front surface accommodation unit 14, and an engagement portion 1413 for facilitating attachment and detachment of the guide unit 141 is also provided. As a result, for example, one-touch attachment and detachment via an adhesive or the like becomes possible, and after the guide unit 141 is detached, the disinfection operation can be easily performed only by wiping the flat surface of the front surface accommodation unit 14. Furthermore, although not illustrated, the above-described odor component capturing portion 1411 and inflow prevention portion 1412 may be provided in the guide unit 141.

Specifically, the user directly sniffs the odor component-containing air K1 linearly released with respect to the nose by the nose cover portion 1414 as much as possible, whereby the odor transfer to the entire guide unit 141 can be prevented. Furthermore, the nose cover portion 1414 can guard the entry of the exhalation K2 from the mouth with the side surface. Furthermore, the exhalation K3 from the nose can be released by the opening 1415 at the distal end of the nose cover portion 1414. Moreover, in a case where the intake unit to be described later generates the airflow K4 containing the odor component in the release direction, the nose cover portion 1414 can also take in the airflow K4 from an opening (not illustrated) on the lower end side of the nose cover portion 1414. The guidance portion 1416 guides the remaining odor component-containing air K5 to the deodorization unit 18, whereby the deodorizing can be easily performed.

### (1-7) Exhaust unit

In a case where the placement drive unit 152 is rotationally driven, the exhaust unit is a portion that exhausts the odor component in the guide unit 141 in conjunction with the rotational drive.

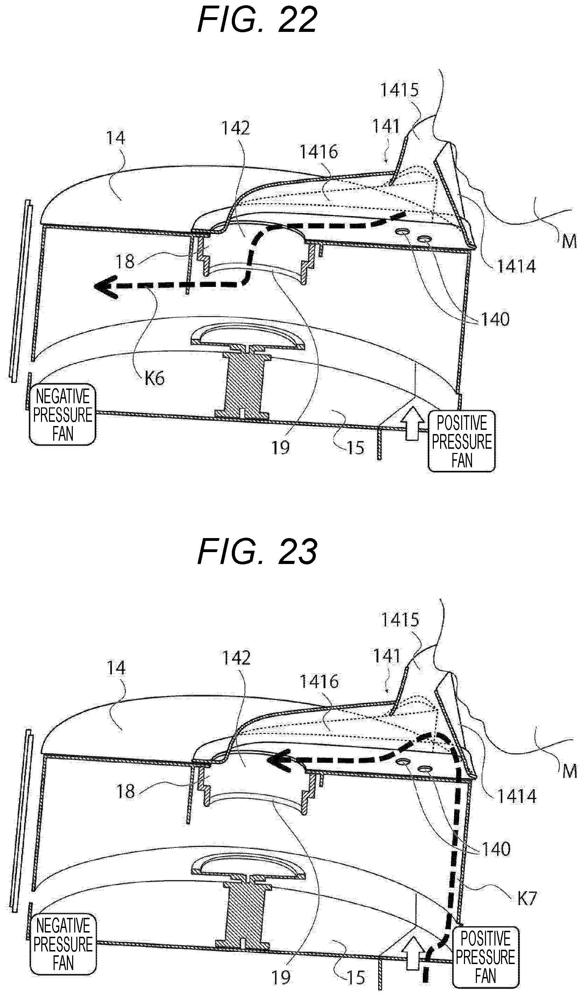

The exhaust unit includes an exhaust fan therein, and discharges the odor component-containing air retained in the guide unit 141 to the outside of the odor generation device 1 in conjunction with the rotational driving operation. As a result, for example, at the same time as the end of the odor provision, the operation of discharging the remaining odor component-containing air can be started, and the unnecessary odor component after the odor provision can be appropriately and quickly removed. Note that, in this case, the duct 19 may be formed at the center of the housing, and the odor component-containing air K6 retained in the guide unit 141 flows into the duct 19 via the guidance portion 1416 of the guide unit 141, the communication hole 142 of the front surface accommodation unit 14, and the deodorization unit 18 in accordance with the operation of the exhaust fan, as illustrated in Fig. 22, and is deodorized.

Note that, in the present embodiment, the number of exhaust units and the number of internal exhaust fans are not particularly limited. An installation position of the exhaust fan is also not particularly limited, and for example, as illustrated in Fig. 22, the exhaust fan can be installed in the back surface accommodation unit 15.

### (1-8) Intake unit

The intake unit is a portion that takes an external airflow into the guide unit 141 in conjunction with the exhaust unit.

The intake unit includes an intake fan therein. In conjunction with the operation of the exhaust unit, the external airflow can also be sucked and deodorized, and more unnecessary odor components can be appropriately removed. The intake fan inside the intake unit is operated by the operation of the exhaust unit, and the external airflow K7 flows in from the side surface of the nose cover portion 1414 of the guide unit 141 as illustrated in Fig. 23. As illustrated in Fig. 23, the external airflow K7 flows into the duct 19 via the guidance portion 1416 of the guide unit 141, the communication hole 142 of the front surface accommodation unit 14, and the deodorization unit 18, and assists deodorization by the exhaust unit.

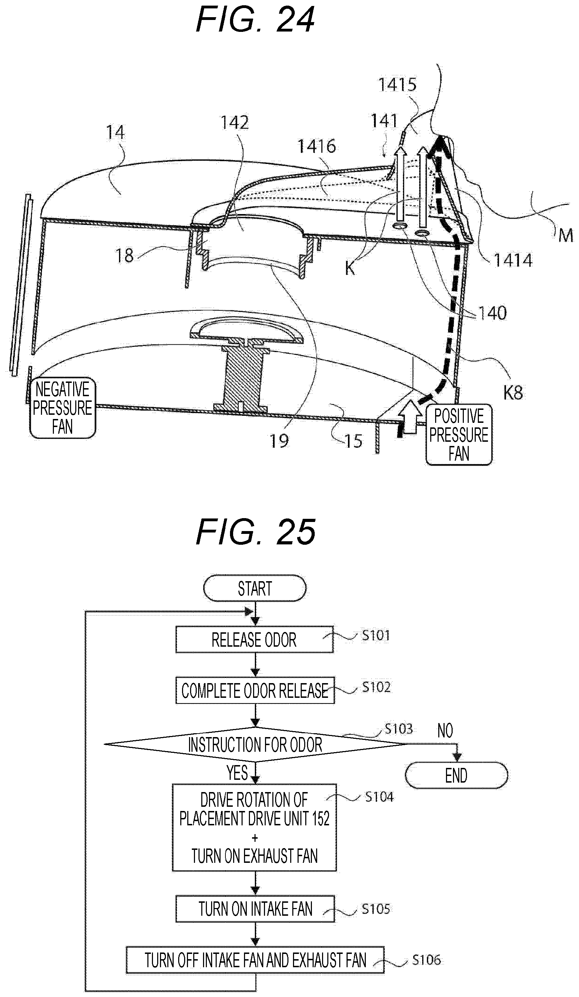

Furthermore, the intake unit can also generate an airflow K8 containing the odor component in the release direction. The airflow K8 flows in from the side surface of the nose cover portion 1414 in accordance with the operation of the intake fan, thereby assisting the operation of delivering the odor component-containing air K released from the release unit 140 to the user. In this case, as illustrated in Fig. 24, the flow passage of the airflow K8 may be designed to be different from the flow passage of the external airflow K7 when assisting deodorization by the exhaust unit.

Note that, in the present embodiment, the number of intake units and the number of internal intake fans are not particularly limited. An installation position of the intake fan is also not particularly limited, and for example, the intake fan can be installed in the back surface accommodation unit 15 as illustrated in Fig. 24.

### (2) Operation example of odor generation device 1

An example of the deodorizing operation of the odor generation device 1 according to the present embodiment will be described below with reference to Fig. 25.

The air K containing a specific odor component is released from the release unit 140, and a specific odor is provided to the user via the guide unit 141 (S101). Next, the release of the air K from the release unit 140 is turned OFF, and the odor release operation is completed (S102).

Next, it is determined whether or not there is a release instruction for another specific odor (S103). In the case of Yes, the process proceeds to step S104. In the case of No, the operation is terminated. Next, in order to release another specific odor, the exhaust fan of the exhaust unit is turned ON in conjunction with the rotational drive of the placement drive unit 152 (S104), and accordingly, the intake fan of the intake unit is turned ON to perform the deodorizing process (S105).

Next, after the deodorizing process in the guide unit 141 is sufficiently performed, the intake fan of the intake unit and the exhaust fan of the exhaust unit are turned OFF (S106). Thereafter, the process returns to step S101 to release another specific odor.

### 3. Third embodiment (odor generation device 1)

### (1) Configuration example of odor generation device 1

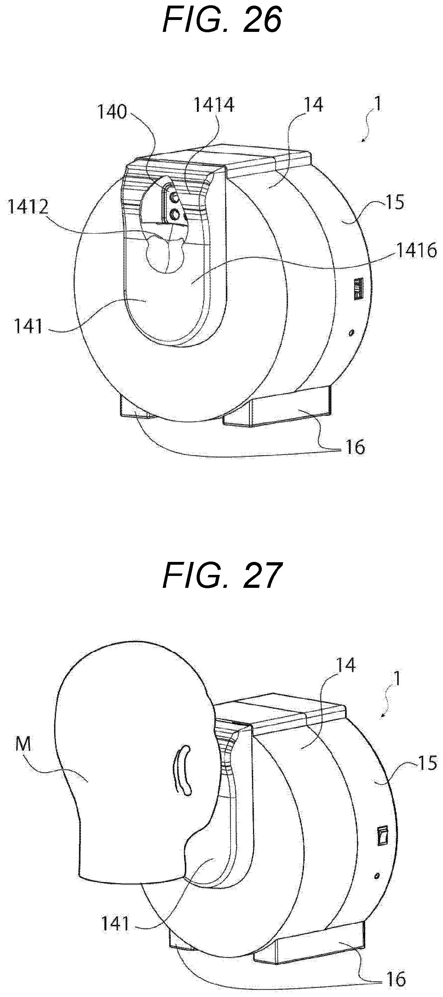

A configuration example of an odor generation device 1 according to a third embodiment of the present technology will be described with reference to Fig. 26. The present embodiment is a modification of the odor generation device 1 according to the first embodiment and the second embodiment described above.

### (1-1) Overall configuration

The odor generation device 1 according to the present embodiment includes the cartridge 10, the holding portion 11, the movable portion 12, the support unit 13, the cartridge unit 20, the front surface accommodation unit 14 including the release unit 140 and the communication hole 142, the guide unit 141, the back surface accommodation unit 15 including the drive mechanism unit 151 and the placement drive unit 152, the position adjustment unit 16, the contact portion 17, the deodorization unit 18, the exhaust unit, the intake unit, and the duct 19. Note that Fig. 26 illustrates a state in which each unit is already engaged with the front surface accommodation unit 14 and/or the back surface accommodation unit 15. Hereinafter, each part will be described in detail.

Note that, in the present embodiment, the cartridge 10, the holding portion 11, the movable portion 12, the support unit 13, the cartridge unit 20, the release unit 140, the communication hole 142, the front surface accommodation unit 14, the drive mechanism unit 151, the placement drive unit 152, the back surface accommodation unit 15, the contact portion 17, the deodorization unit 18, the exhaust unit, the intake unit, and the duct 19 are similar to those described above, and thus the description thereof is herein omitted.

### (1-2) Position adjustment unit 16

In the present embodiment, as illustrated in Fig. 26, by providing the position adjustment unit 16 capable of fixing the position of the guide unit 141 on the side surface of the substantially cylindrical housing, the user can freely adjust the height and angle of the guide unit 141, and can sniff the odor in a comfortable posture. With such a form, as illustrated in Fig. 27, a user M can sniff the odor without bending the neck, and in particular, even a user such as an elderly person who has difficulty in maintaining a posture of bending the neck can sniff the odor in a stable posture. Note that, also in the present embodiment, the position adjustment unit 16 can adjust the position of the housing such that the indwelling portion 202 of the cartridge unit 20 is horizontal at the time of replacement of the cartridge unit 20.

### (1-3) Guide unit 141

In the present embodiment, the guide unit 141 includes a nose cover portion 1414 that covers the nose of the user, an inflow prevention portion 1412 that prevents the inflow of exhalation from the mouth of the user, and a guidance portion 1416 that guides the remaining odor component-containing air to the deodorization unit 18. The inflow prevention portion 1412 may be three-dimensionally formed along the shape of the nose or the mouth as illustrated in Fig. 26. Furthermore, although not illustrated, the odor component capturing portion 1411 and the engagement portion 1413 may be provided in the guide unit 141.

### 4. Fourth embodiment (odor generation device 1)

### (1) Configuration example of odor generation device 1

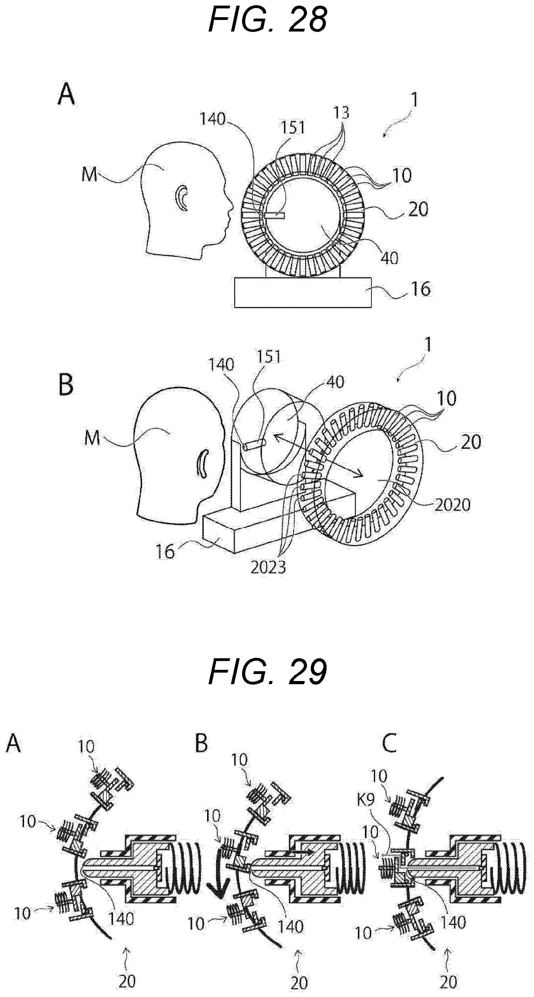

A configuration example of the odor generation device 1 according to the fourth embodiment of the present technology will be described with reference to Fig. 28. The present embodiment is a modification of the odor generation device 1 according to the first embodiment, the second embodiment, and the third embodiment described above.

### (1-1) Overall configuration

The odor generation device 1 according to the present embodiment includes a cartridge 10, a support unit 13, a cartridge unit 20, an accommodation unit 40 including a release unit 140 and a placement drive unit 152, and a position adjustment unit 16. Furthermore, a holding portion 11, a movable portion 12, a guide unit 141, a contact portion 17, a deodorization unit 18, a drive mechanism unit 151, an exhaust unit, an intake unit, a duct 19, and the like may be included as necessary. Hereinafter, each part will be described in detail.

Note that, in the present embodiment, since the cartridge 10, the placement drive unit 152, and the position adjustment unit 16 are similar to those described above, the description thereof is omitted here.

### (1-2) Support unit 13 and cartridge unit 20 for odor generation device

In the present embodiment, the cartridge unit 20 is used in the odor generation device 1 and includes a plurality of cartridges 10 and a plurality of support units 13, and each support unit 13 is engaged with one end of each cartridge 10. The number of the cartridges 10 and the support units 13 included in the cartridge unit 20 can be, for example, 40, but is not limited thereto in the present technology.

In the present embodiment, the cartridge unit 20 radially supports the cartridge 10 via the support unit 13. By radially supporting the cartridge 10, the entire cartridge unit 20 is rotationally driven, and the odor component can be sprayed to the user from one location placed in the vicinity of the release unit 140 to be described later by the placement drive unit 152, and it is possible to avoid deviation of the spray position that may occur when spraying from a plurality of locations. Furthermore, there is also an advantage that it is not necessary to provide a plurality of drive mechanism units 151 and the like to be described later and only one location is required. Moreover, since the entire cartridge unit 20 is rotationally driven, the odor component can be transferred to the odor holding body 104 using the centrifugal force when the odor component is transferred to the odor holding body 104 in the cartridge 10, so that the odor can be efficiently generated. Note that the cartridge unit 20 may radially support the plurality of cartridges 10 in a plurality of planar shapes, in addition to supporting the plurality of cartridges 10 in one planar shape.

In the present embodiment, the form of the indwelling portion 202 is not particularly limited, but for example, the indwelling portion can be formed in a substantially columnar shape or the like which includes a hole 2020 at the center and supports the support units 13 radially placed inside. Furthermore, in the present embodiment, the discharge hole 2023 may be provided at the end of the cartridge 10 on the side not engaged with the support unit 13.

### (1-3) Accommodation unit 40

As illustrated in Fig. 28B, an accommodation unit 40 is provided with the release unit 140 capable of communicating with the discharge hole 2023 of the desired cartridge 10. Odor component-containing air is released to the outside through the release unit 140. In the present embodiment, for example, as illustrated in Fig. 28B, the accommodation unit 40 may be formed to be accommodated inside the hole 2020 of the cartridge unit 20, and in this case, the cartridge unit 20 can have the arrow direction in Fig. 28B as an attaching and detaching direction. The cartridge unit 20 can be rotationally driven by the placement drive unit 152 after being attached to the accommodation unit 40.

### (2) Operation example of odor generation device 1

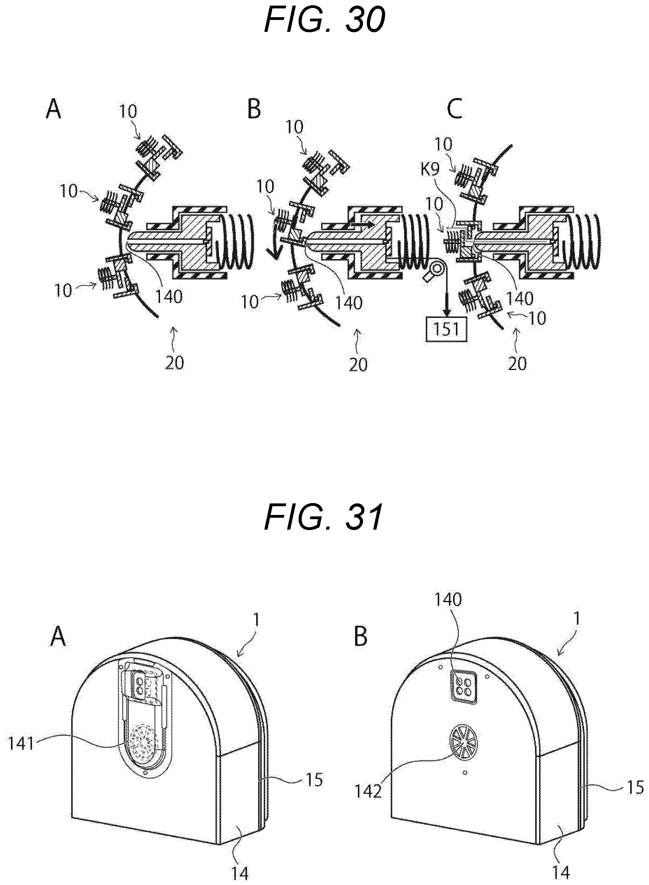

An operation example of the odor generation device 1 according to the present embodiment will be described below with reference to Figs. 29 and 30.

For example, as illustrated in Fig. 29, a pump for releasing the air K9 may be provided on a substantially extended line of the release unit 140 at a timing when the odor component-containing air K9 is released. In this case, in a state where the release of the air K9 is OFF, the state as illustrated in Fig. 29A is set, and when switching to the desired cartridge 10, the cartridge unit 20 is rotationally driven with a rotational force sufficient to climb over the step of the cartridge 10 as illustrated in Fig. 29B, and then, in a state where the release of the air K9 is ON, the desired cartridge 10 and the release unit 140 are engaged and the odor component-containing air is released as illustrated in Fig. 29C.

Furthermore, for example, as illustrated in Fig. 30, a drive mechanism unit 151 for releasing the odor component-containing air K9 may be provided on a substantially extended line of the release unit 140 at a timing when the odor component-containing air K9 is released. In this case, in a state where the release of the air K9 is OFF, the state as illustrated in Fig. 30A is set, and when the cartridge is switched to the desired cartridge 10, the distal end of the release unit 140 is pushed down by the drive mechanism unit 151 as illustrated in Fig. 30B, and in a state where the release of the air K9 is ON, the desired cartridge 10 and the release unit 140 are engaged and the odor component-containing air is released as illustrated in Fig. 30C.

### 5. Fifth embodiment (odor generation device 1)

### (1) Configuration example of odor generation device 1

A configuration example of the odor generation device 1 according to the fifth embodiment of the present technology will be described with reference to Fig. 31. The present embodiment is a modification of the odor generation device 1 according to the first embodiment, the second embodiment, the third embodiment, and the fourth embodiment described above.

### (1-1) Overall configuration

The odor generation device 1 according to the present embodiment includes a cartridge 10, a holding portion 11, a movable portion 12, a support unit 13, a cartridge unit 20, a front surface accommodation unit 14 having a release unit 140, a communication hole 142, an odor control unit 143, and an airflow guidance portion 144, a guide unit 141, a back surface accommodation unit 15 having a drive mechanism unit 151 and a placement drive unit 152, a contact portion 17, a deodorization unit 18, an exhaust unit, an intake unit, and a duct 19. Furthermore, a position adjustment unit 16 and the like may be included as necessary. Note that Fig. 31 illustrates a state in which each unit is already engaged with the front surface accommodation unit 14 and/or the back surface accommodation unit 15. Hereinafter, each part will be described in detail.

Note that, in the present embodiment, the cartridge 10, the holding portion 11, the movable portion 12, the support unit 13, the cartridge unit 20, the release unit 140, the communication hole 142, the drive mechanism unit 151, the placement drive unit 152, the back surface accommodation unit 15, the contact portion 17, the deodorization unit 18, the exhaust unit, the intake unit, and the duct 19 are similar to those described above, and thus the description thereof is omitted here.

### (1-2) Front surface accommodation unit 14 and back surface accommodation unit 15

In the present embodiment, the housing in which the front surface accommodation unit 14 and the back surface accommodation unit 15 are engaged is substantially semi-elliptical columnar. As a result, the user can sniff the odor without bending the neck, and in particular, even a user such as an elderly person who has difficulty in maintaining a posture with a bent neck can sniff the odor in a stable posture. Note that, also in the present embodiment, the position adjustment unit 16 can be provided on the bottom surfaces of the front surface accommodation unit 14 and the back surface accommodation unit 15 as necessary.

### (1-3) Guide unit 141

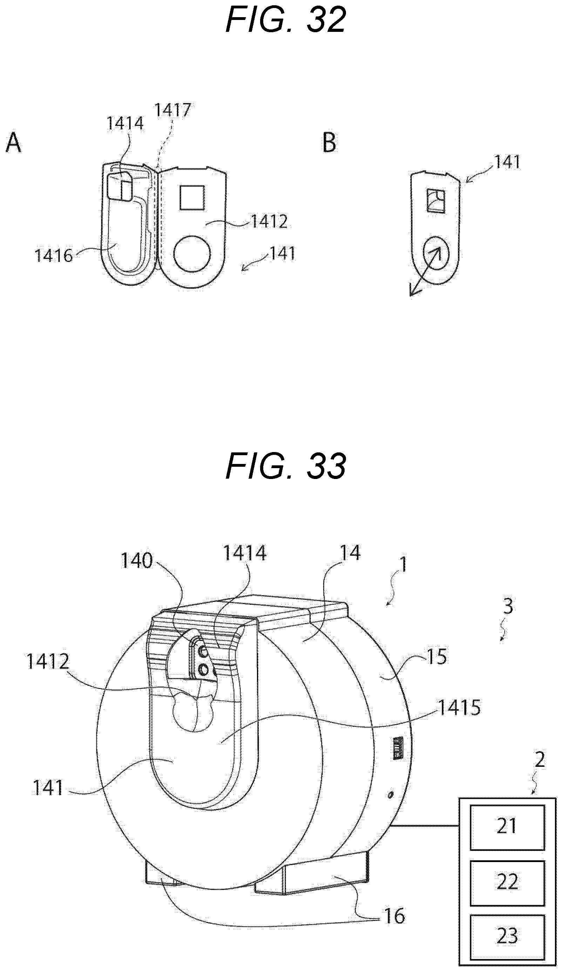

In the present embodiment, as illustrated in Fig. 32, the guide unit 141 includes a nose cover portion 1414 that covers the nose of the user, an inflow prevention portion 1412 that prevents the inflow of exhalation from the mouth of the user, and a guidance portion 1416 that guides remaining odor component-containing air to the deodorization unit 18. Furthermore, although not illustrated, the odor component capturing portion 1411 and the engagement portion 1413 may be provided in the guide unit 141. Moreover, in the present embodiment, the guide unit 141 includes a folded-back portion 1417, whereby the guide unit 141 can be easily formed three-dimensionally, and the folded-back portion 1417 can be stacked and stored in an opened state, so that the flowability, the storability, and the shape maintainability are also improved.

Fig. 32B is a diagram illustrating the guide unit 141 in a state where Fig. 32A is folded back on the basis of the folded-back portion 1417. In this case, the arrow direction in Fig. 32B can be set as the attaching and detaching direction. In the present embodiment, the deodorizing efficiency can be improved, and mixing with the remaining odor component-containing air and adhesion of the air to the guide unit 141 can be reduced. Furthermore, the odor component-containing air can also be efficiently sucked, and the suction error due to the posture, the size of the nose, and the position of the nose can also be reduced.

### (1-4) Odor control unit 143

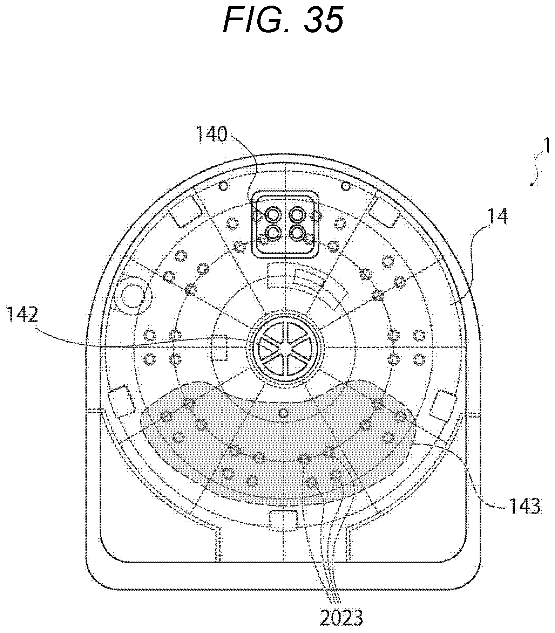

Fig. 35 is a view of the odor generation device 1 according to the fifth embodiment as viewed from a side of the front surface accommodation unit 14. Note that, in Fig. 35, for the sake of explanation, the front surface accommodation unit 14 is illustrated in a transparent manner. In the present embodiment, the front surface accommodation unit 14 has the odor control unit 143 on a part of the inner surface. Since the odor control unit 143 is provided, odor components remaining in the release opening 101b of the cartridge 10, the discharge hole 2023 of the cartridge unit 20, and the like can be removed. Furthermore, the odor component is prevented from rising when the cartridge 10 or the cartridge unit 20 is stored.

The odor control unit 143 may be disposed on any surface as long as the surface is not below the release unit 140. For example, as illustrated in Fig. 35, the odor control unit may be disposed on a surface as far as possible from the release unit 140. Furthermore, in a case where the cartridge unit 20 includes a plurality of the cartridges 10 in which the concentration of the odor component differs stepwise, the odor control unit 143 is preferably placed on the upper surface at least where the cartridge 10 having the highest concentration of the odor component is held. This makes it possible to further prevent the rising of the odor component during storage.

The odor control unit 143 is, for example, a deodorant or an adsorbent, and may be one in which a gas adsorbing substance such as activated carbon is woven or kneaded, or a fiber or a structure itself has a gas adsorption structure. A material forming the odor control unit 143 is similar to that of the odor component capturing portion 1411, and thus is omitted here. Specifically, for example, the deodorant may be a cloth in which a deodorizing agent is incorporated, and may have a cushioning property. Furthermore, for example, the odor control unit 143 may have a plurality of areas for storing granular activated carbon or the like.

Furthermore, in the present embodiment, when the placement drive unit 152 is rotationally driven, the release opening 101b of the cartridge 10 and the discharge hole 2023 of the cartridge unit 20 pass through the lower portion of the odor control unit 143 in accordance with the rotational drive, so that odor components can be automatically wiped off.

### (1-5) Airflow guidance portion 144

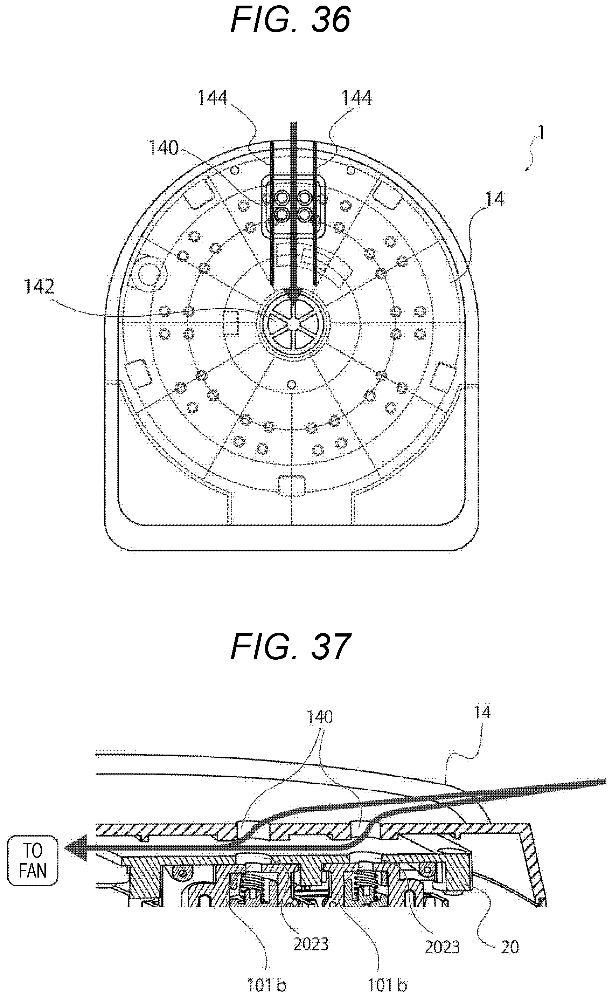

Fig. 36 is a diagram different from Fig. 35 and illustrating the odor generation device 1 according to the fifth embodiment as viewed from the side of the front surface accommodation unit 14. In Fig. 36, for the sake of explanation, the front surface accommodation unit 14 is depicted in a transparent manner. Furthermore, Fig. 37 is a cross-sectional view schematically illustrating a state in which outside air is guided. In the present embodiment, the front surface accommodation unit 14 includes an airflow guidance portion 144 that guides an airflow inward. By including the airflow guidance portion 144, it is possible to function as an intake flow path when the outside air is taken in by the intake fan. By sucking the outside air by the intake fan, as illustrated in Fig. 37, wind is generated in the vicinity of the release opening 101b of the cartridge 10 and the discharge hole 2023 of the cartridge unit 20, and the remaining odor component can be blown off.

As illustrated in Fig. 36, in a case where the outside air is taken in from the release unit 140, the airflow guidance portion 144 is preferably formed so that the outside air can escape from the release unit 140 to the intake fan. As a result, the outside air flowing in from the release unit 140 can be reliably guided to the intake fan in the center of the housing. Specifically, the airflow guidance portion 144 may be a rib or the like formed inside the front surface accommodation unit 14.

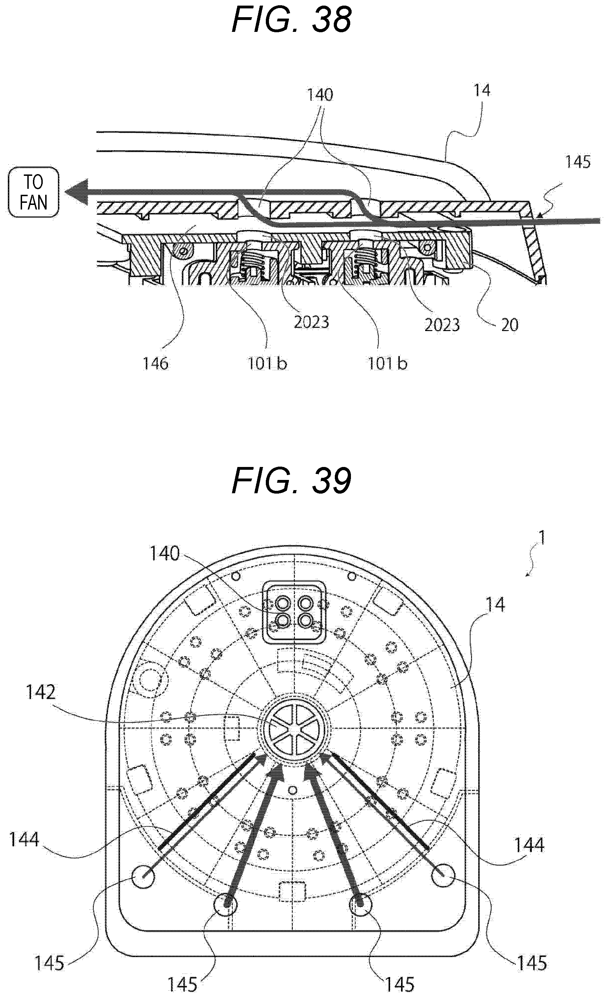

Fig. 38 is a cross-sectional view schematically illustrating a state in which outside air is guided, different from Fig. 37. As illustrated in Fig. 38, in a case where a hole 145 is provided in a side surface, an upper surface, or the like of the front surface accommodation unit 14 to take in outside air from the hole 145, the airflow guidance portion 144 may be formed such that the outside air escapes from the hole 145 to the intake fan. In this case, in addition to the airflow guidance portion 144, a partition 146 or the like may be formed in the front surface accommodation unit 14 as illustrated in Fig. 38.

Fig. 39 is a diagram of the odor generation device 1 according to the fifth embodiment, which is different from Figs. 35 and 36, as viewed from the side of the front surface accommodation unit 14. In Fig. 39, for the sake of explanation, the front surface accommodation unit 14 is depicted in a transparent manner. In the present embodiment, air may be circulated using the internal pressure to generate wind in the vicinity of the release opening 101b of the cartridge 10 or the discharge hole 2023 of the cartridge unit 20. In this case, as illustrated in Fig. 39, holes 145 may be provided in a side surface, an upper surface, or the like of the front surface accommodation unit 14 to circulate air at a high internal pressure. Furthermore, in this case, as illustrated in Fig. 39, the airflow guidance portion 144 may be formed from the hole 145 toward the intake fan. Note that this operation may be set to be performed not only after the odor component is released from the release unit 140 but also before the start of an olfactory examination, olfactory training, olfactory presentation neurodegenerative disease prevention and/or treatment device, an odor experience device, or the like to be described later.

### (2) Operation example of odor generation device 1

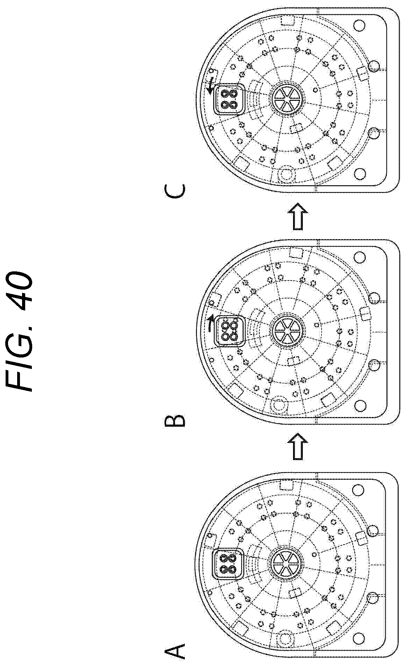

An operation example of the odor generation device 1 according to the present embodiment will be described below with reference to Fig. 40.

The odor generation device 1 according to the present embodiment drives the placement drive unit 152 while the odor component-containing air is released from the release unit 140, and moves the release opening 101b of the cartridge 10 and the discharge hole 2023 of the cartridge unit 20 to the left, right, up, down, or the like, so that the user can feel the odor more strongly.

Specifically, for example, in a case where the placement drive unit 152 is rotationally driven, the release opening 101b of the cartridge 10 and the discharge hole 2023 of the cartridge unit 20 can be moved left and right by a servo motor or the like at a desired rotation angle (for example, 1 to 15°, preferably 2 to 13°) with respect to the minimum release time (for example, 0.1 to 5 seconds) of the odor component-containing air. The driving of the servomotor or the like is preferably performed in a sequence of outputting/stopping the odor component-containing air = ON time of air discharge = period of outputting the odor component-containing air. More specifically, for example, as illustrated in Figs. 40A to C, it is possible to drive to reciprocate one or a plurality of times within a predetermined section using a servomotor or the like within the minimum odor component-containing air discharge time.

### 6. Sixth embodiment (odor generation device 1)

### (1) Configuration example of odor generation device 1

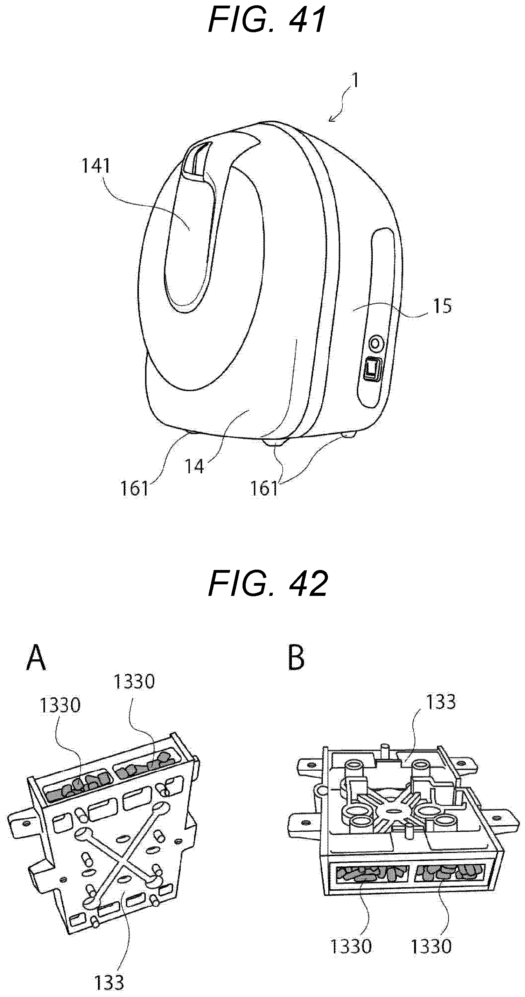

A configuration example of the odor generation device 1 according to the sixth embodiment of the present technology will be described with reference to Fig. 41. The present embodiment is a modification of the odor generation device 1 according to the first embodiment, the second embodiment, the third embodiment, the fourth embodiment, and the fifth embodiment described above.

### (1-1) Overall configuration

The odor generation device 1 according to the present embodiment includes a cartridge 10, a holding portion 11, a movable portion 12, a holder 133, a support unit 13, a cartridge unit 20, a front surface accommodation unit 14 including a release unit 140, a communication hole 142, an odor control unit 143, and an airflow guidance portion 144, a guide unit 141, a back surface accommodation unit 15 including a drive mechanism unit 151 and a placement drive unit 152, a contact portion 17, a deodorization unit 18, an exhaust unit, an intake unit, and a duct 19. Furthermore, a position adjustment unit 16 and the like may be included as necessary. Note that Fig. 41 illustrates a state in which each unit is already engaged with the front surface accommodation unit 14 and/or the back surface accommodation unit 15. Hereinafter, each part will be described in detail. Note that, in the odor generation device 1 of the sixth embodiment, the housing itself may have an inclination of several degrees so that the user can easily bring the nose close to the release hole 120.

Note that, in the present embodiment, the cartridge 10, the holding portion 11, the movable portion 12, the support unit 13, the cartridge unit 20, the release unit 140, the communication hole 142, the odor control unit 143, the airflow guidance portion 144, the front surface accommodation unit 14, the placement drive unit 152, the back surface accommodation unit 15, the contact portion 17, the deodorization unit 18, the exhaust unit, the intake unit, and the duct 19 are similar to those described above, and thus the description thereof is herein omitted.

### (1-2) Holder 133

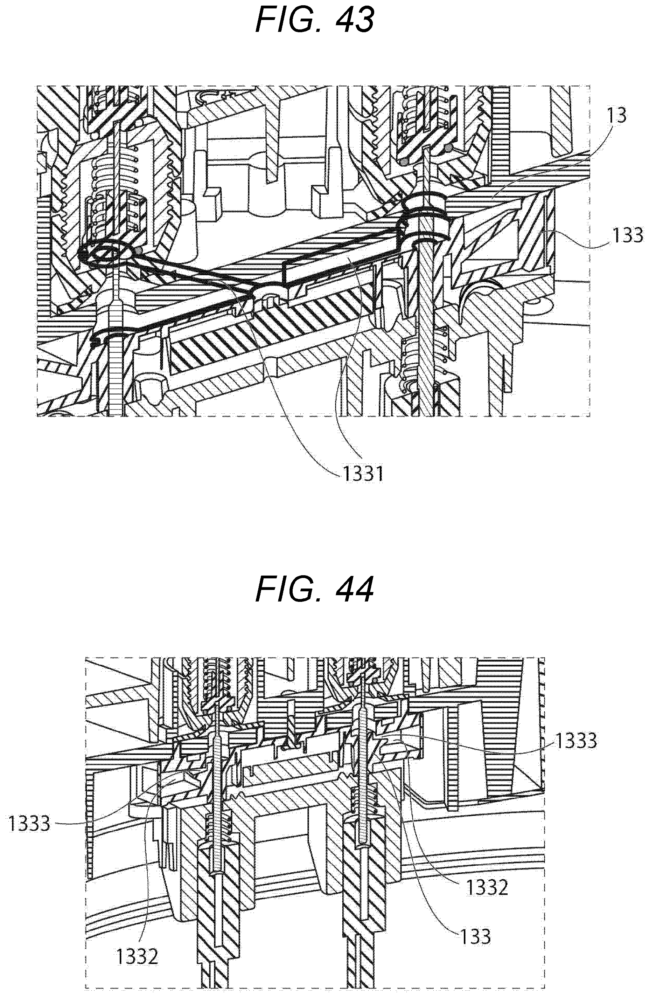

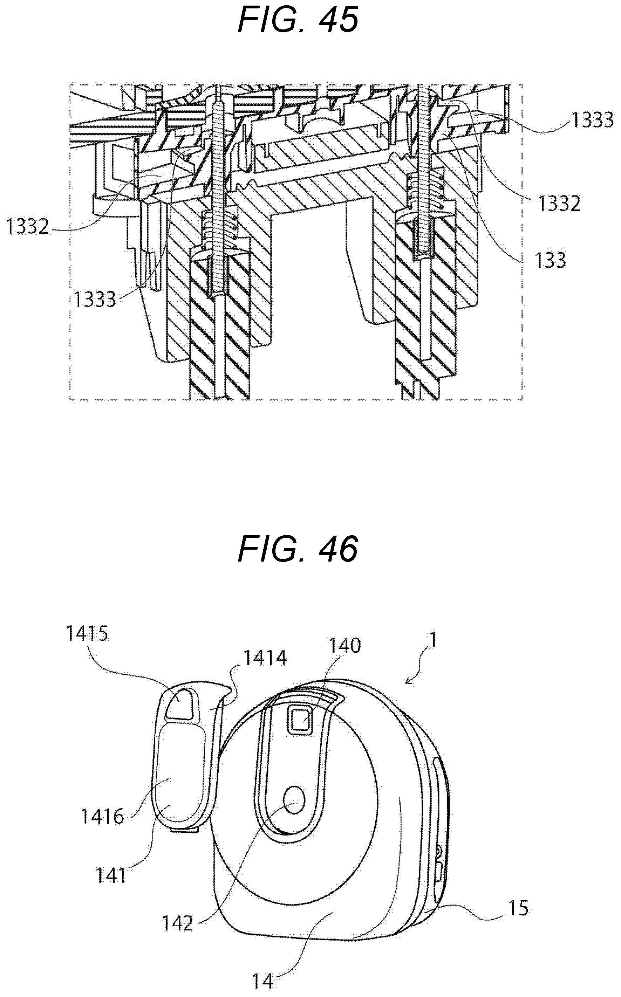

Fig. 42 is a diagram illustrating an example of an embodiment of the holder 133. In the present embodiment, the holder 133 is provided at the lower part of the support unit 13 in a case where the front surface accommodation unit 14 is on the upper side. The holder 133 is provided for each fraction in which an air flow is generated, and includes an intake flow path 1331 (see Fig. 43), an area 1332 for storing a deodorant 1330 (see Figs. 44 and 45), and a flow path 1333 connecting the area 1332 and a ventilation path (see Figs. 44 and 45). As a result, it is possible to prevent the rising of the odor due to the backflow in the discharge direction of the odor component-containing air and the odor residue.

The deodorant 1330 is not particularly limited, but is preferably granular activated carbon. In a case where granular activated carbon is used as the deodorant 1330, a mesh film or the like may be disposed in a part of the flow path 1333 of the holder 133 so that the activated carbon does not flow into each flow path. Examples of the membrane of the mesh material include a nonwoven fabric, a waterproof/breathable membrane or a filter, and a dustproof filter.

### (1-3) Guide unit 141

In the present embodiment, as illustrated in Fig. 46, the guide unit 141 includes a nose cover portion 1414 that covers the nose of the user, an opening 1415 provided on the upper end side of the nose cover portion 1414, and a guidance portion 1416 that guides the remaining odor component-containing air to the deodorization unit 18. In the present embodiment, the guidance portion 1416 has a recess and has a structure in which the user's mouth can be easily accommodated. Furthermore, this makes it easy to store the guide units 141 in an overlapping manner, so that the flowability, the storage property, and the shape maintainability are also improved.

The material forming the guide unit 141 is not particularly limited, but in the present embodiment, the guide unit is preferably formed of a material having a deodorizing effect, and for example, examples thereof include a porous inorganic material such as zeolite (microporous crystalline aluminosilicate) or SUM41; layered inorganic compound such as montmorillonite and smectite (for example, clay minerals and the like); porous coordination polymer (PCP/MOF); tea charcoal; and materials containing copper ions. Furthermore, the material may be paper (including recycled paper), wood, bamboo sheath, plastic, or the like partially containing a naturally-derived porous carbon material (for example, Triporous (registered trademark)), and in the present embodiment, paper partially containing a naturally-derived porous carbon material is preferable.

The guide unit 141 is a part necessary for deodorizing the discharged odoriferous component-containing air, but is preferably disposable each time from the viewpoint of hygiene reasons, odor residue, and the like because a user such as a subject sticks his/her nose. Therefore, at the same time as the guide unit 141 is made of the recycle-based material, the conventional material has a problem of inhibiting the odor component-containing air from which the original odor (for example, the odor of the paper itself and the like) of the material is discharged. Therefore, the guide unit 141 needs to be as odorless as possible. Therefore, by containing a part of a naturally occurring porous carbon material in the conventional material, the guide unit 141 has been successfully made odorless.

### (1-4) Drive mechanism unit 151

In the present embodiment, as will be described later, when an olfactory examination, olfactory training, or the like is actually performed, it is possible to confirm that the shape memory alloy SMA and a drive circuit thereof normally operate before performing the discharge operation of the odor component-containing air. Whether or not the operation is completely performed can be confirmed only by checking the air containing the odor component while performing the discharge operation, and thus, it is impossible to perform an examination without human intervention. However, it is possible to simply confirm whether or not the circuit operation is normally performed by energizing the shape memory alloy SMA. As a result, it is possible to confirm circuit defects such as disconnection and short circuit of the shape memory alloy SMA. This simple check is hereinafter referred to as a "self-test".

Here, since the shape memory alloy SMA controls the valve of the slot in performing the self-test, there is a problem that the valve is opened and the odor component-containing air leaks out when energization is performed in a state where the odor component-containing air enters. Therefore, it is not practical that the odor component-containing air leaks every time the self-test is performed.

In response to this problem, the inventors of the present application turned ON the shape memory alloy SMA only for about 500 ms, and performed the self-test during that time. The shape memory alloy SMA is a device that generates a driving force by heating during energization, that is, since the shape memory alloy SMA operates by a temperature change, there is a time lag of about 1 second from the energization to the generation of the driving force. Therefore, if the power is turned OFF within 1 second after energization, the odor component-containing air is not discharged. A margin of about 500 ms is sufficient to confirm the circuit of the shape memory alloy SMA. As a result, it is possible to realize a self-test without odor leakage.

### (1-5) Bottom convex portion 161

The bottom convex portion 161 does not function as the position adjustment unit 16 as in the above-described embodiment, but is a portion that can adjust the position of the housing so that the indwelling portion 202 is substantially horizontal to the installation surface when the cartridge unit 20 is simply replaced. Therefore, although the bottom convex portion 161 has no function of adjusting the position of the guide unit 141, the cartridge unit 20 can be smoothly replaced by including the bottom convex portion 161.

### 7. Seventh embodiment (olfactory examination or olfactory training system 3)

### (1) Configuration example of olfactory examination or olfactory training system 3

A configuration example of an olfactory examination or olfactory training system 3 according to a seventh embodiment of the present technology will be described with reference to Fig. 33.

### (1-1) Overall configuration

An olfactory examination or olfactory training system 3 according to the present embodiment can be applied to, for example, various olfactory examination methods including a conventionally known olfactory examination method including a T&T olfactometer method, an olfactory examination method used overseas, and a new olfactory examination method that can be developed in the future.

The olfactory examination or olfactory training system 3 according to the present embodiment can be applied to training such as sniffing several types of scents for a predetermined time a predetermined number of times, and can be used for measures such as an odor judge's test and an aromatherapy test.

The olfactory examination or olfactory training system 3 according to the present embodiment includes the above-described odor generation device 1 and the information processing device 2 that instructs the placement drive unit 152. Hereinafter, each part will be described in detail. Note that, in the present embodiment, since the odor generation device 1 is similar to that described above, the description thereof is omitted here.

### (1-2) Information processing device 2

The information processing device 2 is a device that controls the placement drive unit 152. The form of the information processing device 2 is, for example, various devices or the like including the user interface unit 21, and the user can give an instruction to the placement drive unit 152 via the user interface unit 21. Specifically, examples thereof include a tablet terminal, a wearable terminal, a personal computer terminal, and a smartphone.

Furthermore, in a case where the odor generation device 1 further includes the support unit 13 that supports the plurality of cartridges 10, and the support unit 13 supports the plurality of cartridges 10 having the same odor and different concentrations of the odor, the information processing device 2 can instruct the placement drive unit 152 to move the second support unit 13b that supports the plurality of cartridges 10 having the same odor and a concentration of the odor different from the concentration of the plurality of cartridges 10 supported by the first support unit 13a after the first support unit 13a that supports the plurality of cartridges 10 having the same odor and different concentrations of the odor. As a result, for example, it is possible to perform control so as to release the same odor at each concentration of the level 1 having the lowest concentration of the odor to the level 8 having the highest concentration of the odor in a desired procedure, and for example, it is possible to present the odor to the user according to a conventionally known procedure of the olfactory examination or the olfactory training.

The information processing device 2 is connected to the odor generation device 1 via a wired or wireless network. In this case, an instruction may be given to each unit of the odor generation device 1 other than the placement drive unit 152 via the user interface unit 21. Specifically, for example, in a case where an olfactory examination is performed, a doctor, a nurse, a laboratory technician, or the like can instruct to release a specific odor via the user interface unit 21. Furthermore, in this case, a storage unit 22 that stores an answer or the like from the subject who has smelled a specific odor may be included. Furthermore, an analysis unit 23 that performs analysis based on the answer may be included. The storage unit 22 and the analysis unit 23 can also be connected to the odor generation device 1 via a wired or wireless network.

Note that the information processing device 2 may be included in the housing of the odor generation device 1, but may be outside the housing. Furthermore, these are not essential components in the present embodiment, and an external device or the like can also be used.

Note that the functions performed by each unit of the odor generation device 1 according to the present embodiment can be stored as a program in a general-purpose computer or a hardware resource including a control unit including a CPU and the like, a recording medium (for example, a non-volatile memory (for example, a USB memory or the like), an HDD, a CD, or the like), and the like to function. Furthermore, the function may be realized by a server computer or a cloud connected via a wired or wireless network.

### (2) Operation example of olfactory examination or olfactory training system 3

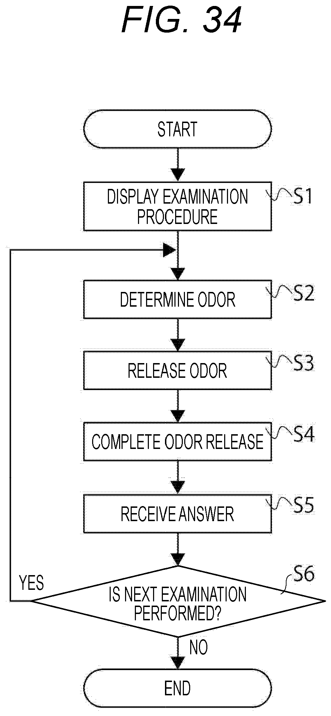

An operation example of the olfactory examination of the olfactory examination or olfactory training system 3 according to the present embodiment will be described below with reference to Fig. 34.

First, the examiner such as a doctor, a nurse, or a laboratory technician instructs the subject to display the examination procedure using the information processing device 2 (S1). In the present embodiment, various devices having the user interface unit 21 may be provided not only on the inspector side but also on the examinee side. Next, the inspector determines an odor (olfactory element) to be released to the subject, and instructs the odor generation device 1 via the information processing device 2. At this time, the placement drive unit 152 that has received the instruction places the cartridge 10 having a specific odor in the vicinity of the release unit 140, and releases the odor according to the operation example of the odor generation device 1 described above (S3).

Next, when the release of the odor is completed (S4), the information processing device 2 receives an answer from the subject, and the storage unit 22 stores the answer (S5). Next, the inspector determines whether or not to perform the next examination (S6), and in the case of Yes, returns to step S2 again and determines the next odor. In a case where the placement drive unit 152 is rotationally driven, when the cartridge 10 having the next specific odor is placed in the vicinity of the release unit 140, the deodorizing operation may be performed according to the operation example of the odor generation device 1 described above. In the case of No, the examination ends as it is.

### 8. Eighth embodiment (olfactory presentation neurodegenerative disease prevention and/or treatment device)

The olfactory presentation neurodegenerative disease prevention and/or treatment device according to the present embodiment includes the above-described odor generation device 1 and an information processing device 2 that instructs the placement drive unit 152. Note that, in the present embodiment, since the odor generation device 1 and the information processing device 2 are similar to those described above, the description thereof is omitted here.

In recent years, in specific neurodegenerative diseases, it is known that olfactory dysfunction occurs prior to deterioration of cognitive function. For example, it is known that in Alzheimer's dementia, deposition of amyloid β protein or phosphorylated tau protein in an olfactory related region is observed prior to atrophy of the hippocampus. Therefore, by using the olfactory presentation neurodegenerative disease prevention and/or treatment device according to the present technology, it is possible to accurately evaluate the olfactory sense of the subject and to use it for prevention and/or treatment of a neurodegenerative disease.

### 9. Ninth embodiment (odor experience device)

The odor experience device according to the present embodiment includes the above-described odor generation device 1 and the information processing device 2 that instructs the placement drive unit 152. Note that, in the present embodiment, since the odor generation device 1 and the information processing device 2 are similar to those described above, the description thereof is omitted here.

By using the odor experience device according to the present embodiment, a conventionally known or new odor can be efficiently presented to the user. The odor experience device according to the present embodiment can be mounted on an automobile, a head mounted display, a relaxation product such as a neck pillow or an eye pillow, or the like. In the case of being mounted on an automobile, for example, an odor can be generated on the basis of an instruction of a driver or a passenger, position information of the automobile, movement of the driver or the passenger, a biological signal, or the like may be detected, and the odor may be generated on the basis of the detection result. In the case of being mounted on a head mounted display, for example, an odor can be generated in conjunction with an image presented on the display, a motion of the user, a biological signal, or the like may be detected, and the odor may be generated on the basis of the detection result. In the case of being mounted on a relaxation product such as a neck pillow or an eye pillow, for example, an odor can be generated on the basis of an instruction of a user, a motion of the user or a biological signal may be detected, and the odor may be generated on the basis of the detection result.

Furthermore, the odor experience device according to the present embodiment may be a device that releases an odor to a wide range of space. Specifically, it can be mounted on a product attracting customers such as a vending machine, a digital signage, and a robot. In the case of being mounted on a customer attracting product, for example, actions, facial expressions, and the like of an unspecified number of users can be detected, and an odor can be generated on the basis of the detection result.

Note that the present technology can also adopt the following configurations.
[1] An odor generation device including:
   a cartridge that holds a tubular body containing an odor component;
   a release unit that releases the odor component to an outside; and
   a placement drive unit that, among a plurality of the cartridges, places a specific cartridge near the release unit.
[2] The odor generation device according to [1], in which the placement drive unit is linearly driven, XY-axis driven, or rotationally driven.
[3] The odor generation device according to [1] or [2], in which the release unit includes a guide unit that guides air containing the odor component near a nose of a user.
[4] The odor generation device according to [3], further including an odor component capturing portion in the guide unit.
[5] The odor generation device according to [3] or [4], further including, in the guide unit, an inflow prevention portion that prevents an inflow of exhalation from a nose and/or a mouth of a user.
[6] The odor generation device according to any one of [3] to [5], further including a position adjustment unit capable of adjusting a position of the guide unit.
[7] The odor generation device according to any one of [3] to [6], further including, in a case where the placement drive unit is rotationally driven, an exhaust unit that exhausts the odor component in the guide unit in conjunction with rotational drive of the placement derive unit.
[8] The odor generation device according to [7], further including an intake unit that takes an external airflow into the guide unit in conjunction with the exhaust unit.
[9] The odor generation device according to [8], in which the intake unit generates an air flow containing the odor component in the release direction.
[10] The odor generation device according to any one of [3] to [9], in which the guide unit is detachable.
[11] The odor generation device according to any one of [1] to [10], further including
   a support unit that supports the plurality of cartridges,
   in which the support unit supports the plurality of the cartridges having the same odor and different concentrations of the odor.
[12] The odor generation device according to [11], in which the placement drive unit rotationally drives each of the support units.
[13] The odor generation device according to [12], in which the placement drive unit moves, after a first support unit that supports the plurality of cartridges having the same odor and different concentrations of the odor, a second support unit that supports the plurality of cartridges having the same odor and concentrations of the odor different from the concentrations of the plurality of cartridges supported by the first support unit.
[14] The odor generation device according to any one of [1] to [13], further including
   a front surface accommodation unit,
   in which the front surface accommodation unit includes an odor control unit on a part of an inner surface.
[15] The odor generation device according to [14], in which an airflow guidance portion that guides an airflow is formed inside the front surface accommodation unit.
[16] The odor generation device according to any one of [1] to [15], further including
   a drive mechanism unit including a pusher that is connected to an operation shaft and pushes a shaft in the cartridge from an outside, and a drive source that drives the pusher,
   in which the drive mechanism unit confirms that a drive circuit operates normally before performing operation of discharging odor component-containing air.
[17] An olfactory examination or olfactory training system including:
   an odor generation device including, a cartridge that holds a tubular body containing an odor component, a release unit that releases the odor component to an outside, and a placement drive unit that, among a plurality of the cartridges, places a specific cartridge near the release unit; and
   an information processing device that controls the placement drive unit.
[18] The olfactory examination or olfactory training system according to [17], further including
   a support unit that supports a plurality of the cartridges,
   in which the support unit supports the plurality of cartridges having a same odor and different concentrations of the odor, and
   the information processing device instructs the placement drive unit to move, after a first support unit that supports the plurality of cartridges having the same odor and different concentrations of the odor, a second support unit that supports the plurality of cartridges having the same odor and concentrations of the odor different from the concentrations of the plurality of cartridges supported by the first support unit.
[19] An olfactory presentation neurodegenerative disease prevention and/or treatment device including:
   an odor generation device including, a cartridge that holds a tubular body containing an odor component, a release unit that releases the odor component to an outside, and a placement drive unit that, among a plurality of the cartridges, places a specific cartridge near the release unit; and
   an information processing device that controls the placement drive unit.
[20] An odor experience device including:
   an odor generation device including, a cartridge that holds a tubular body containing an odor component, a release unit that releases the odor component to an outside, and a placement drive unit that, among a plurality of the cartridges, places a specific cartridge near the release unit; and
   an information processing device that controls the placement drive unit.

### REFERENCE SIGNS LIST

- 1: Odor generation device
- 10: Cartridge
- 101, 101a, 101b: Opening
- 102: Tubular body
- 103: Tubular body holding portion
- 104: Holding body
- 105, 105a, 105b, 105c: Ventilation portion
- 106: Crushing portion
- 107: Pressing portion
- 108: Component guidance portion
- 11: holding portion
- 110: Space
- 111: Communication hole
- 12: Movable portion
- 120: Hole
- 121: Engagement portion
- 13: Support unit
- 13a: First support unit
- 13b: Second support unit
- 130: Communication hole
- 131: Positioning mechanism
- 132: Fixing portion
- 133: Holder
- 1330: Deodorant
- 1331: Intake flow path
- 1332: Area for storing deodorant 1330
- 1333: Flow path connecting area 1332 and ventilation path
- 14: Front surface accommodation unit
- 140: Release unit
- 1401: Engaged portion
- 141: Guide unit
- 1411: Odor component capturing portion
- 1412: Inflow prevention portion
- 1413: Engagement portion
- 1414: Nose cover portion
- 1415: Opening
- 1416: Guidance portion
- 1417: Folded-back portion
- 142: Communication hole
- 143: Odor control unit
- 144: Airflow guidance portion
- 145: Hole
- 146: Partition
- 15: Back surface accommodation unit
- 150: Fitting portion
- 151: Drive mechanism unit
- 152: Placement drive unit
- 16: Position adjustment unit
- 161: Bottom convex portion
- 17: Contact portion
- 171: Screw protrusion
- 172: Screw groove
- 18: Deodorization unit
- 19: Duct
- 20: Cartridge unit for odor generation device
- 202: Indwelling portion
- 2020: Hole
- 2021: Protrusion
- 2022: Positioning mechanism
- 2023: Discharge hole
- 2: Information processing device
- 21: User interface unit
- 22: Storage unit
- 23: Analyzing unit
- 3: Olfactory examination or olfactory training system
- 40: Accommodation unit
- M: User

## Claims

1. An odor generation device comprising:
a cartridge that holds a tubular body containing an odor component;
a release unit that releases the odor component to an outside; and
a placement drive unit that, among a plurality of the cartridges, places a specific cartridge near the release unit.

2. The odor generation device according to claim 1, wherein the placement drive unit is linearly driven, XY-axis driven, or rotationally driven.

3. The odor generation device according to claim 1, wherein the release unit includes a guide unit that guides air containing the odor component near a nose of a user.

4. The odor generation device according to claim 3, further comprising an odor component capturing portion in the guide unit.

5. The odor generation device according to claim 3, further comprising, in the guide unit, an inflow prevention portion that prevents an inflow of exhalation from a nose and/or a mouth of a user.

6. The odor generation device according to claim 3, further comprising a position adjustment unit capable of adjusting a position of the guide unit.

7. The odor generation device according to claim 3, further comprising, in a case where the placement drive unit is rotationally driven, an exhaust unit that exhausts the odor component in the guide unit in conjunction with rotational drive of the placement derive unit.

8. The odor generation device according to claim 7, further comprising an intake unit that takes an external airflow into the guide unit in conjunction with the exhaust unit.

9. The odor generation device according to claim 8, wherein the intake unit generates an airflow containing the odor component in the release direction.

10. The odor generation device according to claim 3, wherein the guide unit is detachable.

11. The odor generation device according to claim 1, further comprising
a support unit that supports the plurality of cartridges,
wherein the support unit supports the plurality of the cartridges having a same odor and different concentrations of the odor.

12. The odor generation device according to claim 11, wherein the placement drive unit rotationally drives each of the support units.

13. The odor generation device according to claim 12, wherein the placement drive unit moves, after a first support unit that supports the plurality of cartridges having the same odor and different concentrations of the odor, a second support unit that supports the plurality of cartridges having the same odor and concentrations of the odor different from the concentrations of the plurality of cartridges supported by the first support unit.

14. The odor generation device according to claim 1, further comprising
a front surface accommodation unit,
wherein the front surface accommodation unit includes an odor control unit on a part of an inner surface.

15. The odor generation device according to claim 14, wherein an airflow guidance portion that guides an airflow is formed inside the front surface accommodation unit.

16. The odor generation device according to claim 1, further comprising
a drive mechanism unit including a pusher that is connected to an operation shaft and pushes a shaft in the cartridge from an outside, and a drive source that drives the pusher,
wherein the drive mechanism unit confirms that a drive circuit operates normally before performing operation of discharging odor component-containing air.

17. An olfactory examination or olfactory training system comprising:
an odor generation device including, a cartridge that holds a tubular body containing an odor component, a release unit that releases the odor component to an outside, and a placement drive unit that, among a plurality of the cartridges, places a specific cartridge near the release unit; and
an information processing device that controls the placement drive unit.

18. The olfactory examination or olfactory training system according to claim 17, further comprising
a support unit that supports a plurality of the cartridges,
wherein the support unit supports the plurality of cartridges having a same odor and different concentrations of the odor, and
the information processing device instructs the placement drive unit to move, after a first support unit that supports the plurality of cartridges having the same odor and different concentrations of the odor, a second support unit that supports the plurality of cartridges having the same odor and concentrations of the odor different from the concentrations of the plurality of cartridges supported by the first support unit.

19. An olfactory presentation neurodegenerative disease prevention and/or treatment device comprising:
an odor generation device including, a cartridge that holds a tubular body containing an odor component, a release unit that releases the odor component to an outside, and a placement drive unit that, among a plurality of the cartridges, places a specific cartridge near the release unit; and
an information processing device that controls the placement drive unit.

20. An odor experience device comprising:
an odor generation device including, a cartridge that holds a tubular body containing an odor component, a release unit that releases the odor component to an outside, and a placement drive unit that, among a plurality of the cartridges, places a specific cartridge near the release unit; and
an information processing device that controls the placement drive unit.
